# EUROPÄISCHE PATENTSCHRIFT

(11) **EP 1 748 975 B1**
(45) Veröffentlichungstag und Bekanntmachung des Hinweises auf die Patenterteilung: **13.02.2008**
(21) Anmeldenummer: 05748062.6
(22) Anmeldetag: 12.05.2005
(51) Int. Cl.: C07C 51/38, C07C 53/126, C07C 55/02, C07B 53/00

(54) **VERFAHREN ZUR HERSTELLUNG OPTISCH AKTIVER 3-ALKYLCARBONSÄUREN UND DEREN ZWISCHENPRODUKTE**
METHOD FOR THE PRODUCTION OF OPTICALLY ACTIVE 3-ALKYL CARBOXYLIC ACIDS AND THE INTERMEDIATE PRODUCTS THEREOF
PROCEDE POUR PRODUIRE DES ACIDES 3-ALKYLCARBOXYLIQUES OPTIQUEMENT ACTIFS ET LEURS PRODUITS INTERMEDIAIRES

(30) Priorität: 27.05.2004 DE 102004025901
(43) Veröffentlichungstag der Anmeldung: 07.02.2007
(73) Patentinhaber: Wacker Chemie AG, 81737 München (DE)
(72) Erfinder: SORGER, Klas, 81371 München (DE); STOHRER, Jürgen, 82049 Pullach (DE)
(74) Vertreter: Fränkel, Robert
(86) Internationale Anmeldenummer: PCT/EP2005/052163
(87) Internationale Veröffentlichungsnummer: WO 2005/115955

(56) Entgegenhaltungen:
- JAMES ASON ET.AL.: "Branched-chain fatty acids. XVI. Synthesis of the optical isomers of 15-methyloctadecanoic acid" J.AM.CHEM.SOC., Bd. 72, 1950, Seiten 4695-4697, XP002343656 in der Anmeldung erwähnt

## Beschreibung

Die Erfindung betrifft ein Verfahren zur Herstellung von optisch aktiven 3-Alkylcarbonsäuren. Optisch aktive 3-Alkylcarbonsäuren sind wichtige Synthesebausteine für die Herstellung von pharmazeutischen Wirkstoffen. Beispielsweise finden (R)-3-Methylheptansäure und (R)-3-Methylhexansäure als Bestandteile und/oder Synthesebausteine von Wirkstoffen zur Immunregulierung Anwendung.

Aus US 5245079 ist ein Verfahren bekannt, bei dem (R)-3-Methylheptansäure und (R)-3-Ethylheptansäure in einer mehrstufigen Synthesesequenz ausgehend von racemischem trans-4-Hexen-3-ol und trans-4-Hepten-3-ol hergestellt werden. Bei diesem Verfahren findet im ersten Syntheseschritt eine Racematspaltung (Sharpless-Reaktion) statt. Die Ausbeute dieses Schritts ist einerseits auf 50 % d. Th. begrenzt, und andererseits erfordern mehrere Reaktionsschritte des Verfahrens Umsetzungen bei Temperaturen von < -60 °C, was das Verfahren insbesondere bei Anwendung im industriellen Maßstab unwirtschaftlich macht.

In JP 03095138 und WO 9322269 werden Racematspaltungsverfahren zur Herstellung von (R)-3-Methylheptansäure und (S)-3-Methylheptansäure durch Kristallisation unter Zuhilfenahme von 1-Phenylethylamin und 1-Phenylpropylamin als chirale Hilfsstoffe beschrieben. Bei beiden Verfahren werden die optisch aktiven Carbonsäuren erst nach mehreren aufeinander folgenden Kristallisationen bzw. Umkristallisationen in ausreichendem Enantiomerenüberschuss von > 97 % ee erhalten. Die geringen Ausbeuten von bis zu < 15 % d. Th. und der hohe verfahrenstechnische Aufwand genügen nicht den Anforderungen eines wirtschaftlichen Prozesses im industriellen Maßstab.

Aus U. Berens, H.-D. Scharf, Synthesis, 1991, S. 832 ist die Herstellung von (S)-4-Benzyloxy-3-methylbutansäure bekannt. Dabei wird (S)-1-Benzyloxypropan-2-ol durch Tosylierung aktiviert, Malonsäuredi-tert-butylester mit dem Tosylat in polar aprotischem Dimethylformamid unter Inversion alkyliert, der alkylierte Malonester hydrolysiert und zur (S)-4-Benzyloxy-3-methylbutansäure decarboxyliert. Die Umsetzung des offenkettigen sekundären Toluolsulfonats von (S)-1-Benzyloxypropan-2-ol mit dem Kaliumsalz von Malonsäuredi-tert.-butylester in Dimethylformamid verläuft jedoch mit partieller Racemisierung und nur 65 % chemischer Ausbeute. Die Gesamtausbeute über alle Stufen beträgt nur 45 %.

Keines der aus dem Stand der Technik bekannten Verfahren liefert optisch aktive 3-Alkylcarbonsäuren in zugleich hohen chemischen und optischen Ausbeuten unter großtechnisch realisierbaren und/oder wirtschaftlich interessanten Bedingungen.

Es bestand somit die Aufgabe, ein alternatives Verfahren zur Herstellung von optisch aktiven 3-Alkylcarbonsäuren bereit zu stellen.

Die Aufgabe wurde gelöst durch ein Verfahren, das von einem preiswerten und großtechnisch verfügbaren enantiomerenreinen Edukt ausgeht, welches im Laufe der Synthese einer vollständig stereoselektiven Inversion ohne Racemisierung bei gleichzeitig hohen Ausbeuten unterworfen wird.

Gegenstand der Erfindung ist ein enantioselektives Verfahren zur Herstellung optisch aktiver 3-Alkylcarbonsäuren (I) enthaltend die folgenden Schritte:
A) Überführung eines optisch aktiven sekundären Alkohols (II) in eine optisch aktive, aktivierte Verbindung (III) durch Einführung einer Abgangsgruppe
B) Umsetzung der aktivierten Verbindung mit einem Malonsäurederivat zu einer optisch aktiven, alkylierten Malonsäureverbindung (IV), wobei die Umsetzung ausschließlich in Gegenwart eines oder mehrerer Lösemittel ausgewählt aus der Klasse der Ether oder der Carbonsäureester erfolgt, wobei optional ein Zusatz an einem oder mehreren aprotisch polaren Lösemitteln oder Alkoholen als Cosolvens erfolgen kann, wobei dieser Zusatz maximal 30% des insgesamt zugesetzten Lösemittelvolumens beträgt
C) gegebenenfalls Verseifung zu der korrespondierenden Säure (V) und
D) abschließende Decarboxylierung der Säure (V) zur optisch aktiven 3-Alkylcarbonsäure als Produkt.

Ein wesentliches Kennzeichen der erfindungsgemäßen Synthesesequenz ist die Tatsache, dass die Umsetzung im Schritt B) durch die Wahl einer geeigneten Klasse an Lösemitteln bzw. Lösemittelgemischen gleichzeitig unter nahezu vollständiger stereoselektiver Inversion und sehr hohen Ausbeuten erfolgt.

Das Lösemittel wird dabei ausschließlich aus einem oder mehreren Lösemitteln ausgewählt aus der Klasse der Ether oder der Carbonsäureester ausgewählt. Optional kann zu den oder dem Lösemitteln aus der Klasse der Ether oder Carbonsäureester ein Zusatz von einem oder mehreren aprotischen polaren Lösemitteln oder Alkoholen als Cosolventien erfolgen, wobei der Anteil des Zusatzes an dem oder den aprotischen polaren Lösemittelen oder Alkoholen 30 % des insgesamt eingesetzten Lösemittelvolumens nicht überschreiten soll, verbunden mit der Maßgabe, dass es sich bei dem optional zugesetzten Cosolvens nicht um Hexamethylphosphorsäuretriamid handelt.

Insbesondere ist Gegenstand der Erfindung ein Verfahren zur Herstellung optisch aktiver 3-Alkylcarbonsäuren der allgemeinen Formel (Ia) oder (Ib), 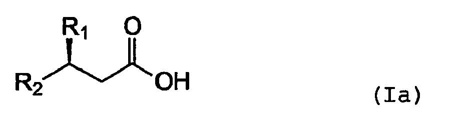 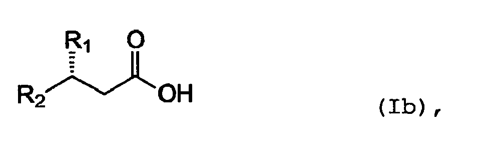 wobei **R¹** und **R²** unabhängig voneinander für einen linearen oder verzweigten, gesättigten oder ungesättigten, cyclischen oder cyclische Gruppen enthaltenden gegebenenfalls mit Q substituierten C₁-C₁₅-Kohlenwasserstoff-Rest stehen
und Q ausgewählt wird aus der Gruppe enthaltend Halogen, Amino, Phthalimido, Hydroxy, Cyano, Nitro, Alkoxy, Aryloxy, Aralkyloxy, Alkylthio, Acyl, Silyl, Silyloxy, Aryl, Heteroaryl,
enthaltend die folgenden Schritte:
A) Umsetzung einer Verbindung der allgemeinen Formel (IIa) oder (IIb) 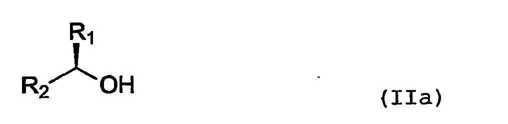 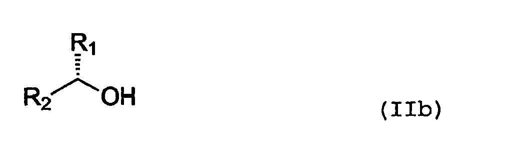 zu aktivierten Verbindungen der allgemeinen Formel (IIIa) oder (IIIb) 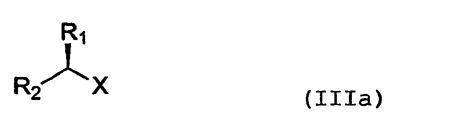 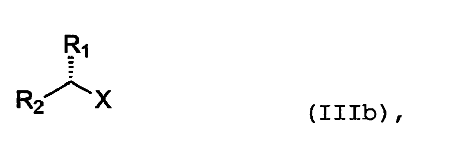 wobei
   **X** ausgewählt wird aus der Gruppe enthaltend Halogen, OSO₂R, OSO₂OR oder OCOR und
   **R** für einen linearen oder verzweigten, gesättigten oder ungesättigten, cyclischen oder cyclische Gruppen enthaltenden C₁-C₁₅-Kohlenwasserstoff-Rest steht
   B) Umsetzung der nach Schritt A) erhaltenen Verbindungen der allgemeinen Formel (IIIa) oder (IIIb) mit einem Malonsäurederivat unter Erhalt von Verbindungen der allgemeinen Formel (IVa) oder (IVb), wobei die Umsetzung ausschließlich in Gegenwart eines oder mehrerer Lösemittel ausgewählt aus der Klasse der Ether oder der Carbonsäureester erfolgt, wobei optional ein Zusatz an einem oder mehreren aprotisch polaren Lösemitteln oder Alkoholen als Cosolvens erfolgen kann, wobei dieser Zusatz maximal 30% des insgesamt zugesetzten Lösemittelvolumens beträgt und 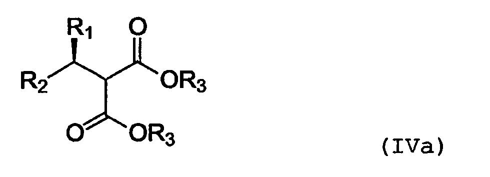 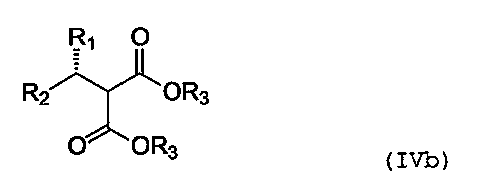 wobei
      **R³** in der allgemeinen Formel (IVa) oder (IVb) jeweils unabhängig voneinander für Wasserstoff oder einen linearen oder verzweigten, gesättigten oder ungesättigten, cyclischen oder cyclische Gruppen enthaltenden C₁-C₁₅-Kohlenwasserstoff-Rest, einen C₁-C₂₀-Trialkyl- oder C₁-C₂₀-Triaryl-Silyl-Rest steht,
   C) gegebenenfalls Verseifung der nach Schritt B) erhaltenen Verbindungen der allgemeinen Formel (IVa) oder (IVb) unter Erhalt von Dicarbonsäuren der allgemeinen Formel (Va) oder (Vb) 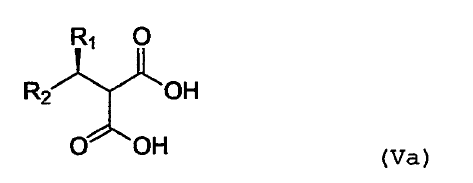 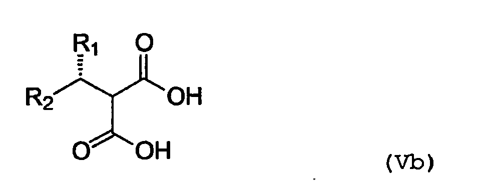
   D) und abschließende Decarboxylierung der nach Schritt C) erhaltenen Dicarbonsäure der allgemeinen Formel (Va) oder (Vb).

So lässt sich nach dem erfindungsgemäßen Verfahren insbesondere (R)-3-Methylhexansäure ausgehend von (S)-2-Pentanol gemäß dem nachfolgenden Reaktionsschema erhalten:

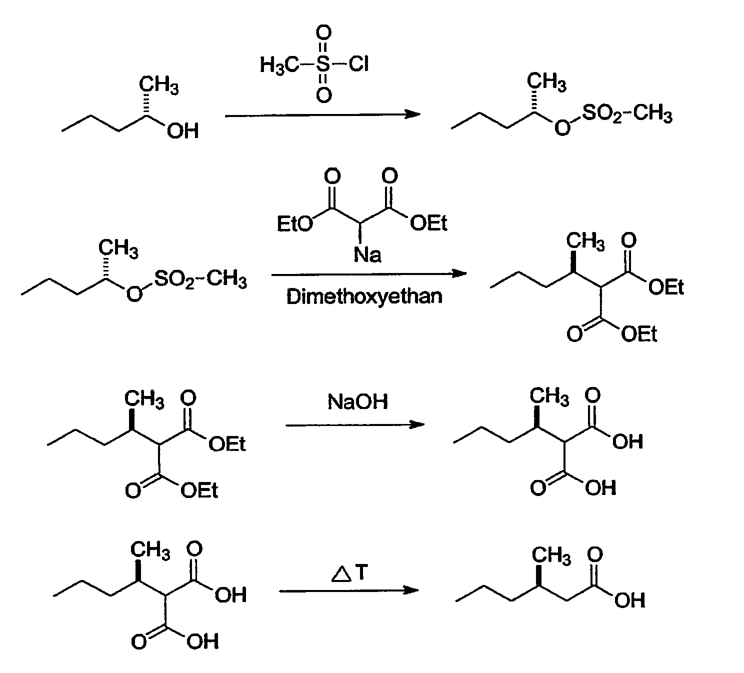

Mögliche Ausführungsformen für die C₁-C₁₅-Kohlenwasserstoffreste **R¹, R²** und **R³** sind lineare oder verzweigte, cyclische oder cyclische Gruppen enthaltende, gesättigte oder ungesättigte C₁-C₁₅-Alkyl-, C₂-C₁₅-Alkenyl- oder C₂-C₁₅-Alkinylreste.

Die C₁-C₁₅-Kohlenwasserstoffreste für **R¹** und **R²** können gegebenenfalls zusätzlich mit **Q** substituiert sein, wobei **Q** ausgewählt werden kann aus der Gruppe enthaltend F, Cl, Br, J, CN, NO₂, OH, Phthalimido, C₁-C₁₀-Alkoxy-, C₁-C₆-Trialkylsilyl-, C₁-C₆-Trialkylsilyloxy-, NH₂, C₁-C₁₀-Alkylamino-, C₁-C₆-Trialkyl-silylamino-, C₁-C₆-Trialkylsilyl-C₁-C₁₀-Alkylamino-, Aralkyloxy-, N-(Aralkyl)amino-, N,N-(Diaralkyl)amino-, Aryl-, Aralkyl-, Alkaryl-, Aralkenyl-, Alkenylaryl- oder Heteroarylreste, wobei letztere ihrerseits substituiert sein können durch Reste ausgewählt aus der Gruppe F, Cl, Br, J, CN, NO₂, C₁-C₁₀-Alkoxyreste, C₁-C₁₀-Alkylaminoreste oder C₁-C₁₀-Alkylreste.

Besonders bevorzugte Reste für **R¹, R²** und **R³** wie auch für **R** werden unabhängig voneinander ausgewählt aus der Gruppe enthaltend Methyl, Ethyl, n-Propyl, Isopropyl, n-Butyl, sec.-Butyl, tert.-Butyl, n-Pentyl, n-Hexyl, n-Heptyl, Cyclopropyl, Cyclobutyl, Cyclopentyl, Cyclohexyl, Allyl, Vinyl, Benzyl, Benzyloxymethyl, Benzyloxyethyl, Benzyloxypropyl, N-(Benzylamino)methyl, N-(Benzylamino)ethyl, N,N-(Dibenzylamino)methyl, N,N-(Dibenzylamino)ethyl, Phthalimidomethyl oder Phthalimidoethyl.

Besonders bevorzugte Substituenten **Q** sind Fluor, Chlor, Brom, Nitro, Cyano, Hydroxy, Amino, N-Methylamino, N,N-Dimethylamino, N-Benzyl, N,N-Dibenzyl, Phthalimido, N-Acetylamino, N-Acetyl-N-methylamino, N-Benzyloxycarbonylamino, Methoxy, Ethoxy, Phenoxy, Benzyloxy, Acetyl, Propionyl, Phenyl, Naphthyl, Benzyl, Furyl, Piperidinyl, Pyrrolidinyl, Quinolinyl, Pyridyl, Piperazinyl, Imidazolyl, Pirimidinyl, Oxazolyl, Isoxazolyl, Morpholinyl, Thiazolyl, Isothiazolyl, Indolyl, Triazinyl, Thienyl oder Thiophenyl, die wiederum durch Reste ausgewählt aus der Gruppe enthaltend Methyl, Ethyl, n-Propyl, Isopropyl, Butyl, sec.-Butyl, tert.-Butyl, Cyclopropyl, Cyclobutyl, Cyclopentyl, Cyclohexyl, Allyl, Vinyl, Phenyl, Furyl, Piperidinyl, Pyrrolidinyl, Quinolinyl, Pyridyl, Piperazinyl, Imidazolyl, Pirimidinyl, Oxazolyl, Isoxazolyl, Morpholinyl, Thiazolyl, Isothiazolyl, Indolyl, Triazinyl, Thienyl, Thiophenyl, Fluor, Chlor, Brom, Nitro, Cyano, Amino, Hydroxy, Methoxy, Ethoxy, Phenoxy, Trimethylsilyl, Triethylsilyl, Acetyl, Propionyl, Amino, N,N-Dimethylamino, N-Benzyl, N-Acetylamino, N-Acetyl-N-methylamino oder N-Benzyloxycarbonylamino substituiert sein können sowie Acetyl-, Amino-, N-Methylamino-, N,N-Dimethylamino-, N-Benzyl-, N-Acetylamino-, N-Acetyl-N-methylamino-, N-Benzyloxycarbonylamino-, Nitro-, Methyl-, Ethyl-, Propyl-, Isopropyl-, Butyl-, sec.-Butyl-, tert.-Butyl-, Methoxy-, Ethoxy-, Phenoxy-, Acetoxy-, Benzyloxy-, Trimethylsilyl-, Trimethylsilyloxy, Triethylsilyloxy-, Fluor-, Chlor-, Brom-, Jod- und Cyanophenyl oder -naphthyl.

Die optisch aktiven Alkohole (Edukte), insbesondere solche der allgemeinen Formel (IIa) oder (IIb), sind kommerziell in enantiomerenreiner oder enatiomerenangereicherter Form verfügbar und können insbesondere durch enantioselektive Reduktion der entsprechenden prochiralen Ketone in Gegenwart von Enzymkatalysatoren wirtschaftlich und großtechnisch in enantiomerenreiner Form erhalten werden (T. C. Rosen, T. Daussmann, J. Stohrer, Chem. Spec., 2004, April, S. 39).

Durch biokatalytische Reduktion hergestellte Alkohole, insbesondere solche der allgemeinen Formel (IIa) oder (IIb), können das zu deren Herstellung eingesetzte korrespondierende prochirale Keton als Verunreinigung enthalten. Es wurde überraschend gefunden, dass dieses Keton die Herstellung der aktivierten Verbindungen durch Einführung einer Abgangsgruppe, besonders bei solchen aktivierten Verbindungen der allgemeinen Formel (IIIa) oder (IIIb), insbesondere bei Sulfonsäureestern, in keiner Weise negativ beeinflusst. Vielmehr fungiert das Keton als Co-Solvens und bildet keine unerwünschten Nebenprodukte oder Verunreinigungen. Nach erfolgter Umsetzung der Alkohole zu den aktivierten Verbindungen, insbesondere Sulfonsäureestern in Schritt A) lassen sich die Ketone problemlos durch Destillation, Kristallisation oder Extraktion abtrennen.

Der Einsatz von mit Keton geringfügig verunreinigten Alkoholen als Edukte ohne vorangehende Aufreinigung macht das Verfahren besonders wirtschaftlich, so dass in einer besonders bevorzugten Ausführungsform des erfindungsgemäßen Verfahrens mit Keton, insbesondere dem des eingesetzten Alkohols korrespondierenden Keton, verunreinigte Alkohole eingesetzt werden. Insbesondere werden Alkohole eingesetzt, die als direkte Verfahrensprodukte aus einem biokatalytischen Herstellungsverfahren für enantiomerenreine oder enatiomerenangereicherte sekundäre Alkohole stammen, insbesondere aus dem oben zitierten im Stand der Technik bekannten Verfahren.

In Schritt A) werden die Alkohole, insbesondere solche der allgemeinen Formel (IIa) oder (IIb) unter Erhalt der Konfiguration in aktivierte Verbindungen, insbesondere solche der allgemeinen Formel (IIIa) oder (IIIb), durch Einführung einer Abgangsgruppe überführt. Methoden zur Aktivierung von Alkoholen als Substrate für die nukleophile Substitution sind dem Fachmann wohlbekannt.

In diesen aktivierten Verbindungen, insbesondere solchen der allgemeinen Formel (IIIa) oder (IIIb), wird die Abgangsgruppe bzw. X im allgemeinen ausgewählt aus der Gruppe enthaltend Halogen, Sulfonsäureester OSO₂R, Schwefelsäureester OSO₂OR und Carbonsäureester OCOR. Bei den aktivierten Verbindungen, insbesondere solchen der allgemeinen Formel (IIIa) oder (IIIb), handelt es sich bei der Abgangsgruppe bzw. bei X bevorzugt um Halogenide ausgewählt aus Chlor, Brom oder Iod oder um Sulfonsäureester OSO₂R, wobei R hier bevorzugt aus Fluor, C₁-C₁₅-Alkyl oder C₁-C₂₀-Aryl-Resten ausgewählt wird, wobei letztere wiederum gegebenenfalls durch Halogen-, Nitro- oder C₁-C₁₅-Alkyl-Reste substituiert sein können. Im Falle von C₁-C₁₅-AlkylResten für R kann R hier aus den oben genannten besonders bevorzugten Ausführungsformen für R ausgewählt werden.

Besonders bevorzugte Abgangsgruppen in den aktivierten Verbindungen bzw. Reste X sind Sulfonsäureester OSO₂R, von denen besonders solche bevorzugt sind, bei denen R für Methyl, Trifluormethyl, Phenyl oder 4-Methylphenyl, insbesondere für Methyl oder 4-Methylphenyl, steht.

Die Einführung einer Sulfonsäureester-Abgangsgruppe bzw. der Reste X gleich OSO₂R in die Alkohole als Edukte unter Erhalt der aktivierten Verbindungen, insbesondere solchen der allgemeinen Formel (IIIa) oder (IIIb), erfolgt im allgemeinen durch Umsetzung der Alkohole mit Sulfonsäurechloriden, insbesondere in Gegenwart einer Base wie beispielsweise aus R. W. Bates, T. R. Devi, Tetrahedron Lett., 1995, 36, S. 509 bekannt.

Als Sulfonsäurechloride werden bevorzugt Methansulfonsäurechlorid, Trifluormethansulfonsäurechlorid, Benzolsulfonsäurechlorid und Toluolsulfonsäurechlorid, insbesondere Methansulfonsäurechlorid und Toluolsulfonsäurechlorid verwendet.

Als Basen finden insbesondere Aminbasen, besonders bevorzugt Triethylamin und Pyridin Verwendung.

Die Reaktion wird in inerten Lösemitteln wie beispielsweise Methylenchlorid, Diethylether, tert.-Butylmethylether oder Toluol durchgeführt.

Die Temperatur bei der Umsetzung gemäß Schritt A) beträgt üblicherweise -50 bis +50 °C, insbesondere -20 bis +30 °C.

Es hat sich bewährt, den Alkohol der allgemeinen Formel, insbesondere solche der allgemeinen Formel (IIa) oder (IIb), mit dem Sulfonsäurechlorid und gegebenenfalls dem Amin im Molverhältnis 1 : (1 bis 2) : (1 bis 4), insbesondere 1 : (1 bis 1.3) : (1 bis 1.7), umzusetzen.

Die hergestellten aktivierten Verbindungen, insbesondere die Sulfonsäureester werden nach der Aufarbeitung, insbesondere durch Hydrolyse, direkt weiterverarbeitet. Es ist auch möglich, diese vor der weiteren Umsetzung in einem zusätzlichen Schritt durch Destillation oder Kristallisation zu reinigen.

In Schritt B) werden die nach Schritt A) erhaltenen aktivierten Verbindungen, insbesondere solche der allgemeinen Formel (IIIa) oder (IIIb), mit einem Malonsäurederivat zu alkylierten Malonsäureverbindungen, insbesondere solchen Verbindungen der allgemeinen Formel (IVa) oder (IVb), unter vollständig stereoselektiver Inversion der Konfiguration umgesetzt.

Es wurde überraschend gefunden, dass die Herstellung der alkylierten Malonsäureverbindungen, insbesondere solchen der allgemeinen Formel (IVa) oder (IVb) - entgegen der vorherrschenden Meinung im Stand der Technik - unter vollständig stereoselektiver Inversion der Konfiguration ohne Racemisierung am Stereozentrum mit sehr hohen optischen Ausbeuten von > 99 % und gleichzeitig sehr hohen chemischen Ausbeuten von bis zu > 90 % verläuft, wenn die Alkyierung in einem oder mehreren Lösemitteln ausgewählt aus der Klasse der Ether oder Carbonsäureester durchgeführt wird, wobei optional ein Zusatz an einem oder mehreren aprotisch polaren Lösemitteln oder Alkoholen als Cosolvens erfolgen kann, wobei dieser Zusatz maximal 30% des insgesamt zugesetzten Lösemittelvolumens beträgt.

Vorzugsweise beträgt der zugesetzte Anteil der polar aprotischen Lösemitteln als Coselventien bezogen auf die Summe der Volumina der insgesamt eingesetzten Lösemittel weniger als 25% , insbesondere weniger als 20%.

In einer besonders bevorzugten Ausführungsform der Umsetzung im Schritt B) wird die Reaktion ausschließlich in einem oder mehreren der Lösemittel ausgewählt aus der Klasse der Ether oder Carbonsäureester durchgeführt ohne dass weitere polar aprotischen Lösemittel oder Alkohole zugesetzt oder zugegen sind, insbesondere in nur einem einzigen Lösemittel ausgewählt aus der Gruppe der Ether.

Ether im Sinne des erfindungsgemäßen Schritts B) sind allgemien solche Verbindungen, die der Fachmann unter diesen Begriff subsumieren würde, also Verbindungen, die durch ein allgemeines Strukturelement R-O-R gekennzeichnet sind, wobei R hier für einen beliebigen organischen Rest steht. Ether im Sinne dieser Erfindung sind demnach insbesondere einfache oder gemischte Dialkyl-, Diaryl-, Alkylaryl-Ether, cyclische Ether, geminale Dialkoxyverbindungen (Acetale), Glycolether, Polyether und/oder Ethoxylate.

Es ist bekannt, dass die Substitution von Malonsäureester ohne Racemisierung verläuft, wenn die Alkylierung des Malonsäureesters unter milden Reaktionsbedingungen, d. h. bei niedriger Reaktionstemperatur durchgeführt wird. In diesem Fall sind stark aktivierende Abgangsgruppen wie beispielsweise Trifluormethansulfonat oder Nitrobenzolsulfonat notwendig. Die Verwendung von Trifluormethansulfonat als Abgangsgruppe zur Alkylierung von Malonat bei einer Temperatur zwischen 0 °C und Raumtemperatur ist beispielsweise aus EP 684240 bekannt. Für die industrielle Anwendung dieser Verfahren nachteilig ist, dass die zur Herstellung der aktivierten Sulfonsäureester notwendigen Sulfonsäurehalogenide oder -anhydride sehr teuer sind, was diese Verfahren für die industrielle Anwendung unwirtschaftlich macht.

Zur Vermeidung der teuren, stark aktivierenden Abgangsgruppen, wie z. B. Trifluormethansulfonat, wurden Verfahren vorgeschlagen, bei denen die billigeren, aber wenig aktivierenden Abgangsgruppen Methansulfonat oder Toluolsulfonat eingesetzt werden. Die Substitutionsreaktion muss wegen der geringen aktivierenden Wirkung dieser Sulfonate aber bei erhöhter Reaktionstemperatur in polar aprotischen, aktivierenden Lösemitteln wie z. B. Dimethylformamid, Dimethylsulfoxid oder Hexamethylphosphorsäuretriamid (HMPT) durchgeführt werden. Unter diesen Reaktionsbedingungen tritt jedoch eine erhebliche Racemisierung ein. Zudem begünstigen polar aprotische, aktivierende Lösemittel Nebenreaktionen, was letzlich zu schlechteren Ausbeuten und niedrigeren Reinheiten der hergestellten Produkte führt.

So ist aus T. Sato, J. Otera, J. Org. Chem., 1995, 60, S. 2627 bekannt, dass die Substitution offenkettiger sekundärer Methansulfonate durch das Natriumsalz von Diethylmalonat in polar aprotischem Dimethylformamid als Lösemittel mit beträchtlicher Racemisierung verläuft. Zur Vermeidung der Racemisierung wird von Sato et. al. ein Verfahren vorgeschlagen, bei dem die Umsetzung von Methansulfonat mit Malonsäurediethylester in Dimethylformamid unter nichtbasischen, neutralen Bedingungen in Gegenwart von Cäsiumfluorid durchgeführt wird. Für das Methansulfonat von (S)-2-Oktanol als Edukt werden mit diesem Verfahren aber lediglich Ausbeuten von 53 bis 60 % erreicht. Aus diesem Grund und der Tatsache, dass ein teures Hilfsreagenz in Form von Cäsiumfluorid verwendet werden muss, ist dieses Verfahren ebenfalls für die industrielle Anwendung wenig geeignet.

Aus U. Berens, H.-D. Scharf, Synthesis, 1991, S. 832 ist weiter bekannt, dass die Umsetzung des offenkettigen sekundären Toluolsulfonats von (S)-1-Benzyloxypropan-2-ol mit dem Kaliumsalz von Malonsäuredi-tert.-butylester in Dimethylformamid mit partieller Racemisierung in einer optischen Ausbeute von 97 % und nur 65 % chemischer Ausbeute verläuft. Für Malonsäurediethylester wurde bei analoger Vorgehensweise sogar nur eine chemische Ausbeute von 44 % erreicht. Mit Malonsäuredimethylester wurden überwiegend polymere Esterkondensationsprodukte erhalten.

Aus M. Larcheveque, Y. Petit, Synthesis, 1991, S. 162 ist weiterhin bekannt, dass die Alkylierung von Di-tert-butylmalonat mit Toluolsulfonsäure- oder Nitrobenzolsulfonsäureestern von α-Hydroxyestern im Lösemittelgemisch Tetrahydrofuran und Hexamethylphosphorsäuretriamid (HMPT) bei Raumtemperatur nach 48 h unter stereospezifischer Inversion mit 80 % Ausbeute verläuft. Um unter diesen milden Bedingungen ausreichende Reaktivität zu erzielen, muss in Gegenwart des stark aktivierenden, polar aprotischen Lösemittels HMPT gearbeitet werden. Dabei wurden aber nur mäßige chemische Ausbeuten von 61 bis 80 % erzielt. Für die industrielle Anwendung dieses Verfahrens sind jedoch die langen Reaktionszeiten, die giftigen Eigenschaften von HMPT und die niedrigen Ausbeuten von nur 60 bis 80 % als größte Nachteile zu nennen. Auch dieses Verfahren ist deshalb für die industrielle Anwendung wenig geeignet.

Das erfindungsgemäße Verfahren zeichnet sich insbesondere dadurch aus, dass auf den Zusatz von problematischen HMPT als Cosolvens verzichtet wird.

Aus J. Uenishi, M. Hamada, Chem. Pharm. Bull., 50, 2002, S. 697 ist weiterhin bekannt, dass 1-(2-Pyridinyl)ethyl-methansulfonat mit Malonsäureester in einer Mischung aus 6 Teilen Tetrahydrofuran und 4 Teilen des aktivierenden polar aprotischen Lösemittels Dimethylsulfoxid unter Inversion substituiert wird. Die Ausbeute der Umsetzung betrug aber nur 75 bis 82 %.

Aus J. M. Brown, J. E. MacIntyre, J. Chem. Soc., Perkin Trans II, 1985, S. 961 ist ferner die Alkylierung des Natriumsalzes von Dimethylmalonat mit (S)-Toluolsulfonsäure-(1-methylpropyl)ester in Methanol als Lösemittel bekannt. Die optische Ausbeute wurde von Brown et al. nicht bestimmt. Allerdings verläuft die Alkylierung von Malonat in dem polar protischen Lösemittel Methanol sowie in Ethanol und Isopropanol unter partieller Racemisierung (siehe Vergleichsbeispiel 2).

Wird hingegen die Umsetzung der aktivierten Verbindungen, insbesondere solchen enthaltend eine Sulfonsäureester-Abgangsgruppe, insbesondere solchen der allgemeinen Formel (IIIa) oder (IIIb), mit X gleich OSO₂R, wobei R insbesondere für Methyl, Phenyl oder 4-Methylphenyl steht, mit Malonsäurederivaten gemäß Schritt B) in einem oder mehreren Lösemitteln aus der Klasse der Ether oder Carbonsäureester in Gegenwart von polar aprotischen Lösemitteln oder Alkoholen durchgeführt, wobei der Anteil des oder der zugesetzten aprotischen polaren Lösemittel oder Alkohole einen Anteil von 30 %bezogen auf die Summe der Volumina der insgesamt eingesetzten Lösemittel nicht übersteigt, verläuft die Herstellung der alkylierten Malonsäureverbindungen, insbesondere solchen der allgemeinen Formel (IVa) oder (IVb), selbst bei erhöhter Temperatur von 85 °C überraschender Weise unter vollständig stereoselektiver Inversion der Konfiguration und ohne Racemisierung mit optischen Ausbeuten von > 99 % und überraschender Weise auch gleichzeitig sehr hohen chemischen Ausbeuten von bis zu > 90 % d. Th. bei nahezu vollständigem Umsatz. Zudem erfolgt die stereospezifische Alkylierung der Malonsäureverbindungen überraschender Weise ohne merkliche Bildung von Nebenprodukten mit sehr hoher Reinheit der hergestellten Produkte.

Beispielsweise verläuft die erfindungsgemäße Umsetzung von enantiomerenreinem (S)-Methansulfonsäure-(1-methylpentyl)ester von > 99.9 % ee mit dem Natriumsalz von Malonsäurediethylester unter Bildung von [(R)-1-Methylpentyl]malonsäurediethylester in 1,2-Dimethoxyethan vollständig stereospezifisch ohne Racemisierung mit > 99.9 % ee und zugleich sehr hoher chemischer Ausbeute von 98 % bei einer Reaktionszeit von lediglich 5 h unter sehr hoher Raum-Zeitausbeute.

Mit den aus dem Stand der Technik bekannten Verfahren verläuft die Umsetzung jedoch mit einem erheblichen Maß an Racemisierung und deutlich schlechteren chemischen Ausbeuten von lediglich 44 bis 82 %.

Führt man konkret die vorgenannte Umsetzung in Dimethylformamid als Lösemittel unter den von U. Berens, H.-D. Scharf, Synthesis, 1991, S. 832 beschriebenen Bedingungen durch, dann erhält man [(R)-1-Methylpentyl]malonsäurediethylester nicht nur mit verminderter chemischer Ausbeute von < 40 % und erheblichem Anteil an Verunreinigungen, sondern aufgrund partieller Racemisierung mit deutlich reduziertem Enantiomerenüberschuss von nur 96.3 % ee (siehe Vergleichsbeispiel 1). Auch in den Lösemitteln Methanol, Ethanol oder Isopropanol findet unter sonst identischen Bedingungen partielle Racemisierung statt (siehe Vergleichsbeispiele 2).

Dies ist umso überraschender, als - entgegen der vorherrschenden Meinung im Stand der Technik - auch ohne Zusatz aktivierender polar aprotischer Lösemittel oder aktivierender Abgangsgruppen trotz hoher Reaktionstemperaturen von bis über 80 °C vollständige Inversion am Stereozentrum ohne Racemisierung stattfindet. Zugleich werden die Reaktionsprodukte in hohen chemischen Ausbeuten und in hoher Reinheit bei kurzen Reaktionszeiten auf sehr wirtschaftliche Weise hergestellt. Besonders überraschend ist, dass die stereospezifische Alkylierung in Lösemitteln aus der Klasse der Ether oder Carbonsäureester selbst in Gegenwart von polar aprotischem Lösemittel oder Alkohol, wobei der Anteil des oder der zugesetzten aprotischen polaren Lösemittel einen Anteil von 30% bezogen auf die Summe der Volumina der insgesamt eingesetzten Lösemittel nicht übersteigt, auch bei Temperaturen von bis über 80 ° mit gegenüber dem Stand der Technik sehr hohen Ausbeuten bis typischerweise > 90 % und gleichzeitig sehr hohen Reinheiten der gebildeten Produkte verläuft.

Die Umsetzung gemäß Schritt B) kann im allgemeinen unter sauren, neutralen oder basischen Bedingungen durchgeführt werden, bevorzugt unter basischen Bedingungen.

Das Malonsäurederivat wird ausgewählt aus der Gruppe enthaltend Malonsäure, Malonsäuremonoester, Malonsäurediester, bevorzugt Malonsäuredialkylester und -bis(trialkylsilylester), insbesondere Malonsäuredimethyl- und -diethylester Verwendung.

Das Malonsäurederivat kann entweder in Form eines mindestens in 2-Position deprotonierten Salzes eingesetzt oder in Gegenwart einer Base umgesetzt werden.

Bevorzugt werden als Malonsäurederivat Alkalisalze der Malonsäuredialkylester und -bis(trialkylsilylester) eingesetzt, insbesondere das Natrium- oder Kaliumsalz von Malonsäuredimethyl- und -diethylester. Es ist auch möglich, das Alkalisalz oder Erdalkalisalz der Malonsäure oder eines Halbesters einzusetzen. Auch können gemischte Salze der Malonsäure oder eines Halbesters eingesetzt werden.

Die Alkalisalze der Malonsäureester werden nach für den Fachmann bekannten Verfahren hergestellt. Insbesondere werden das Natrium- oder Kaliumsalz von Malonsäurediethyl- oder -dimethylester hergestellt, indem man eine Natrium- oder Kaliumbase in einem Lösemittel vorlegt und hierzu den Malonsäureester zudosiert, gegebenenfalls gelöst in einem Lösemittel. Es ist auch möglich, die Natrium- oder Kaliumbase, gegebenenfalls gelöst in einem Lösemittel, zu dem Malonsäureester zuzufügen, gegebenenfalls gelöst in einem Lösemittel. Als Natrium- oder Kaliumbase können im allgemeinen Natrium- oder Kaliumhydroxid, -alkoxid oder -hydrid Verwendung finden, insbesondere Natrium- oder Kaliumalkoxid und -hydrid, besonders bevorzugt -alkoxid. Als Natrium- oder Kaliumalkoxid finden Natrium- oder Kaliummethylat, -ethylat, -isopropylat oder -tert.-butanolat bevorzugt Verwendung.

Als Lösemittel für die Herstellung der Salze der Malonsäurederivate eignen sich insbesondere Alkohole und Ether. In einer bevorzugten Ausführungsform des erfindungsgemäßen Verfahrens werden die Salze ebenfalls in dem für die Umsetzung nach Schritt B) gewählten Lösemittel aus der Klasse der Ether hergestellt. Die Umsetzung findet bei Temperaturen zwischen -50 und 150 °C, insbesondere zwischen 10 und 80 °C, statt.

Gegebenenfalls wird aus der Reaktionsmischung gebildetes Wasser oder Alkohol abdestilliert. Es ist auch möglich, das Reaktionslösemittel nach Herstellung des Malonsäuresalzes durch Destillation zu entfernen und durch ein anderes Lösemittel, bevorzugt aus der Klasse der Ether, zu ersetzen.

Zur Herstellung des Salzes des Malonsäuredialkylesters hat es sich bewährt, den Malonsäuredialkylester mit der Base, insbesondere mit der Natrium- oder Kaliumbase, im Molverhältnis (0.7 bis 2) : 1 umzusetzen. Bevorzugt ist ein Molverhältnis von (1 bis 1.3) : 1.

Nach erfolgter Herstellung des basischen Malonsäurealkalisalzes erfolgt die Umsetzung mit der aktivierten Verbindung, insbesondere solche enthaltend Sulfonsäureester-Abgangsgruppen bzw. Verbindungen der allgemeinen Formel (IIIa) oder (IIIb), worin X für OSO₂R steht, durch Zusammenführen der Komponenten, gegebenenfalls gelöst in einem Lösemittel. Hierzu wird die aktivierte Verbindung zu dem Malonatsalz zugegeben oder das Malonatsalz zu der aktivierten Verbindung, gegebenenfalls jeweils gelöst in einem Lösemittel.

Als Lösemittel für die stereospezifische Herstellung der alkylierten Malonsäureverbindungen, insbesondere denen der allgemeinen Formel (IVa) oder (IVb), die unter vollständiger Inversion am Stereozentrum verläuft, werden nach der erfindungsgemäßen Methode Lösemittel aus der Klasse der Ether oder Carbonsäureester eingesetzt gegebenenfalls mit einem Zusatz an polar aprotischem Lösemittel oder Alkohol von bis zu 30% bezogen auf die Summe der Volumina der insgesamt zugesetzten Lösemittel.

Insbesondere werden für die Umsetzung mit dem Malonsäurederivat gemäß Schritt B) Lösemittel ausgewählt aus der Gruppe enthaltend cyclische Ether, insbesondere Tetrahydrofuran, 2-Methyltetrahydrofuran, 2,5-Dimethyltetrahydrofuran, 2,5-Dimethoxytetrahydrofuran oder 1,4-Dioxan; offenkettige Di- und Oligoether mit mindestens einer Ethylen-Brücke (Glyme-Verbindungen), insbesondere Dimethoxyethan, Diethoxyethan, Diethylenglykol-dimethylether, Diethylenglykol-diethylether, Diethylenglykol-dipropylether, Diethylenglykol-dibutylether, Triethylenglykol-dimethylether, Triethylenglykol-diethylether oder Triethylenglykol-dibutylether.

Bevorzugt für die Umsetzung mit dem Malonsäurederivat gemäß Schritt B) sind auch Lösemittel aus der Klasseder Carbonsäureester ausgewählt aus der Gruppe enthaltend Ameisensäure-, Essigsäure-, Propionsäure-, Buttersäure-, Valeriansäure-methyl-, -ethyl-, -propyl-, -isopropyl-, -butyl-, -isobutyl-, -tert.-butyl-, pentyl-, -hexyl-, -benzylester, insbesondere Essigsäure-, Propionsäure-, Buttersäure-methyl-, - ethyl-, -propyl-, -isopropyl-, -butyl-ester; besonders bevorzugt sind hierbei Essigsäure-ethyl-, -propyl-, -isopropyl- und/oder -butylester.

Die Umsetzung kann in Gegenwart von bis zu 30 Volumen-Teilen bezogen auf die Summe der Volumina der insgesamt eingesetzten Lösemittel an polar aprotischem Lösemittel oder Alkohol als Cosolvens durchgeführt werden.

Als polar aprotische Lösemittel eignen sich grundsätzlich alle dem Fachmann bekannten Lösemittel dieses Typs. Polar aprotische Lösemittel kennzeichnen sich durch hohes Solvatationsvermögen für Kationen und/oder Anionen, Dielektrizitätskonstanten ε von 20 bis >100 und Dipolmomente µ zwischen 2.5 und 6 Debye. Als polar aprotische Lösemittel werden insbesondere Nitrile, Carbonsäureamide, Harnstoffderivate und Sulfoxide verwendet, bevorzugt Acetonitril, Propionitril, Butyronitril, Formamid, N-Methylformamid, Dimethylformamid, Diethylformamid, Dimethylacetamid, Diethylacetamid, N-Methylpyrrolidon, 1,3-Dimethyl-3,4,5,6-tetrahydro-2(1H)-pyrimidinon und Dimethylsulfoxid.

In dem erfindungsgemäßen Verfahren erfolgt explizit kein Zusatz von Hexamethylphosphorsäuretriamid (HMPT) als polar aprotisches Cosolvens.

Neben polar aprotischen Lösemitteln eignen sich auch Alkohole als Cosolvenzien, insbesondere Methanol, Ethanol, Propanol, Isopropanol, Butanol, Isobutanol, tert.-Butanol, Pentanol, Amylalkohol, besonders bevorzugt sind Methanol, Ethanol und Isopropanol.

Neben polar aprotischen Lösemitteln und Alkoholen eignen sich als Cosolvenzien grundsätzlich auch Amine wie z. B. Triethylamin, N,N,N',N'-Tetramethylethylendiamin, Pyridin, Dimethylanilin, Ketone wie z. B. Aceton, Methylethylketon, und halogenierte Kohlenwasserstoffe wie z. B. Dichlorethan, Trichlorethan, Dichlorpropan, Trichlorpropan, Tetrachlormethan, oder deren Mischungen.

Es können auch Gemische zweier oder mehrerer der vorstehend genannten Lösemittel eingesetzt werden.

Besonders bevorzugte Lösemittel aus der Klasse der Ether werden ausgewählt aus der Gruppe enthaltend Tetrahydrofuran, 2-Methyltetrahydrofuran, 2,5-Dimethyltetrahydrofuran, 1,4-Dioxan, Dimethoxyethan, Diethoxyethan, Diethylenglykol-dimethylether, Diethylenglykol-diethylether oder Triethylenglykol-dimethylether. Besonders bevorzugt sind 1,4-Dioxan, 1,2-Dimethoxyethan, 1,2-Diethoxyethan oder Diethylenglykol-dimethylether.

Besonders bevorzugte Lösemittel aus der Klasse der Carbonsäureester werden ausgewählt aus der Gruppe enthaltend die Methyl-, Ethyl-, Propyl-, Isopropyl- oder Butyl-Ester der Essig-, Propion- oder, Buttersäure. Besonders bevorzugt sind Essigsäure-ethyl-, -propyl-, -isopropyl- und/oder -butylester.

Besonders bevorzugte polar aprotische Cosolvenzien sind Acetonitril, Propionitril, Butyronitril, Formamid, Dimethylformamid, Dimethylacetamid und Dimethylsulfoxid.

In einer besonders bevorzugten Ausführungsform beträgt der Anteil der zugesetzten Cosolventien weniger als 20% bezogen auf die Summe der Volumina der insgesamt eingesetzten Lösemittel, insbesondere wird auf den Zusatz eines polar aprotischen Lösemittels oder eines Alkohols als Cosolvens vollständig verzichtet.

In einer besonders bevorzugten Ausführungsform des erfindungsemäßen Verfahrens wird der Schritt B) ausschließlich in Ethern, insbesondere in nur einem Ether oder ausschließlich in Carbonsäureestern, insbesondere in nur einem Carbonsäureester durchgeführt, wobei die Ether und Carbonsäureester jeweils aus den vorgenannten bevorzugten Ausführungsformen für diese Lösemittelklassen ausgewählt werden.

Die Umsetzung gemäß Schritt B) findet bei Temperaturen zwischen 10 und 200 °C, insbesondere zwischen 40 und 140 °C, besonders bevorzugt zwischen 60 und 100 °C, statt.

Die Umsetzung kann auch in Gegenwart eines Phasentransferkatalysators durchgeführt werden.

Es hat sich bewährt, das Malonsäurederivat mit der aktivierten Verbindung, insbesondere solchen der Klasse der Sulfonsäureester im Molverhältnis (0.7 bis 2) : 1 umzusetzen. Bevorzugt ist ein Molverhältnis von (1.0 bis 1.3) : 1.

Nach beendeter Umsetzung ist es möglich, zuerst einen Teil des Lösemittels durch Destillation, gegebenenfalls unter reduziertem Druck, zu entfernen und zurückzugewinnen. Nach beendeter Umsetzung erfolgt Aufarbeitung der Reaktionsmischung, bevorzugt durch wässrige saure oder basische Hydrolyse, gegebenenfalls in Gegenwart eines weiteren Lösemittels. Das erhaltene Produkt, insbesondere eines der allgemeinen Formel (IVa) oder (IVb) kann optional in einem zusätzlichen Schritt durch Destillation, Extraktion oder Chromatographie gereinigt werden.

In einer bevorzugten Ausführungsform des erfindungsgemäßen Verfahrens wird das Rohprodukt, das wegen des hohen erzielbaren Umsatzes bereits in sehr reiner Form gewonnen wird, ohne weitere Aufreinigung direkt weiterverarbeitet.

In Schritt C) werden die nach Schritt B) erhaltenen alkylierten Malonsäureverbindungen, insbesondere solche der allgemeinen Formel (IVa) oder (IVb) unter Bildung der Verbindungen der korrespondierenden Säuren, insbesondere solchen der allgemeinen Formel (Va) oder (Vb), unter vollständigem Erhalt der optischen Konfiguration verseift.

Wird als Malonsäurekomponente Malonsäure selbst, ein Salz der Malonsäure oder ein Malonsäuresilylester, wie beispielsweise Malonsäurebis(trimethylsilylester), eingesetzt, dann erfolgt bei wässriger Aufarbeitung gegebenenfalls direkt in Schritt B) Hydrolyse zu den korrespondierenden Säuren. Eine separate Verseifung in Schritt C) ist in diesen Fällen nicht notwendig.

Wird als Malonsäurekomponente ein Malonsäureester, Malonsäuremonoester oder ein Salz dieser Verbindungen eingesetzt, dann erfolgt in Schritt C) die Esterverseifung.

Die Verseifung der alkylierten Malonsäureverbindungen, insbesondere Malonsäureestern der allgemeinen Formel (IVa) oder (IVb), wiederum insbesondere der Malonsäuredimethyl- und -diethylester, erfolgt unter wässrigen Bedingungen, neutral, sauer oder basisch, bevorzugt unter basischen Bedingungen nach dem Fachmann bekannten Methoden.

Zur Hydrolyse unter basischen Bedingungen werden die nach Schritt B) erhaltenen alkylierten Malonsäureverbindungen, gegebenenfalls in Gegenwart von Lösemittel, mit wässriger Base umgesetzt. Als Basen sind alle hierfür geeigneten, dem Fachmann wohlbekannten Basen geeignet, insbesondere Alkali- und Erdalkalihydroxid, -oxid und -alkoholat, bevorzugt Natrium- und Kaliumhydroxid.

Die Verseifung erfolgt bei Temperaturen zwischen 0 und 150 °C, bevorzugt 40 und 100 °C.

Die Umsetzung kann einphasig, gegebenenfalls in Gegenwart von Lösemittel, insbesondere Alkohol, bevorzugt Methanol und Ethanol, oder zweiphasig, gegebenenfalls in Gegenwart von Lösemittel, durchgeführt werden.

Die Verseifung wird bevorzugt in Gegenwart von Methanol und Ethanol und direkt durch wässrige Base ohne Zugabe von weiterem Lösemittel durchgeführt. Wird kein weiteres Lösemittel verwendet und wird die Verseifung im alkalischen wässrigen Medium durchgeführt, dann gestaltet sich die Umsetzung durch Erzielung hoher Raum-Zeit-Ausbeuten besonders wirtschaftlich.

Nach vollständiger Verseifung erfolgt Aufarbeitung und Isolierung der Produkte der korrespondierenden Säuren, insbesondere solchen der allgemeinen Formel (Va) oder (Vb).

Die Aufarbeitung erfolgt bevorzugt durch Zugabe einer Säure und Extraktion mit organischem Lösemittel. Es kann aber auch nach anderen, dem Fachmann bekannten Verfahren aufgearbeitet werden.

Ein weiterer Gegenstand der Erfindung sind die Verbindungen der allgemeinen Formel (Va) oder (Vb) 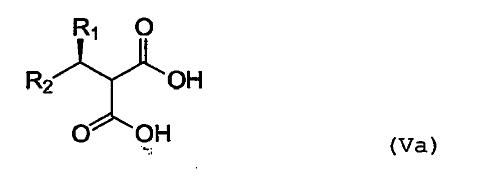 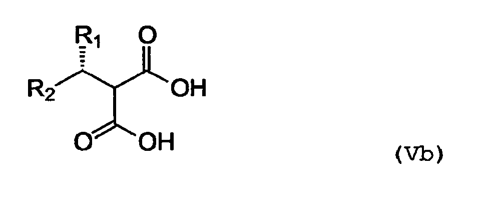 wobei
**R¹** und **R²** die oben genannten Bedeutungen haben, insbesondere aus den oben genannten bevorzugten Ausführungsformen ausgewählt werden.

Die Verbindungen der allgemeinen Formel (Va) oder (Vb) sind als Zwischenprodukte über das erfindungsgemäße Verfahren in enatiomerenreiner bzw. enantiomerenangereicherter Form zugänglich.

Die Verbindungen der allgemeinen Formel (Va) oder (Vb) stellen wertvolle Synthesezwischenprodukte des erfindungsgemäßen Verfahrens dar, da sich diese in einem optionalen zusätzlichen Schritt besonders gut reinigen lassen und so eine Erhöhung der chemischen und optischen Ausbeute (Erhöhung des Enantiomerenüberschusses) der gewünschten Endprodukte möglich wird.

So ist ein weiterer Gegenstand der Erfindung ein Verfahren zur Enantiomerenanreicherung von Verbindungen der allgemeinen Formeln (VIa) oder (Vb) durch Kristallisation oder Umkristallisation.

Die Verbindungen der allgemeinen Formel (Va) oder (Vb) sind im allgemeinen Feststoffe, die sich durch Kristallisation bzw. Umkristallisation einfach reinigen lassen, wobei insbesondere die optische Reinheit verbessert werden kann. So ist es beispielsweise für Alkohole der allgemeinen Formel (IIa) oder (IIb) (Edukte), die nur mit einer vergleichsweise geringen optischer Reinheit von < 95 % ee erhalten werden können, möglich, den Enantiomerenüberschuss und die chemische Reinheit in einem optionalen zusätzlichen Schritt nach Schritt C) gezielt durch Kristallisation zu verbessern. Somit sind auch vergleichsweise kostengünstige - weil nur in geringer optischer Reinheit zugänglich - Edukte, ohne vorherige zusätzliche und aufwändige Enantiomerenanreicherung ebenfalls nach dem erfindungsgemäßen Verfahren erfolgreich umsetzbar.

So wurde beispielsweise überraschenderweise gefunden, dass [(R)-1-Methylbutyl]malonsäure und [(R)-1-Methylpentyl]malonsäure in einem Schritt durch Umkristallisation aus Hexan als Lösemittel in hoher chemischer Ausbeute von > 90 % von 95 % ee auf > 99 % ee angereichert werden können.

Aus J. Cason, R. A. Coad, J. Am. Chem. Soc., 1950, 72, S. 4695 ist bekannt, dass die Herstellung von [(S)-1-Methylbutyl]malonsäure aus (R)-2-Pentanol durch Aktivierung in Form von (R)-2-Brompentan, Alkylierung von Diethylmalonat mit (R)-2-Brompentan in Ethanol und Hydrolyse des Diesters zu [(S)-1-Methylbutyl]malonsäure mit 10 bis 15 % Racemisierung verläuft. Wegen der zu niedrigen optischen Reinheit der [(S)-1-Methylbutyl]malonsäure von bestenfalls 90 % ee konnte eine Enantiomerenanreicherung durch Kristallisation nicht durchgeführt werden, da bevorzugt das Racemat kristallisierte. Nach dem erfindungsgemäßen Verfahren ist es nun erstmals möglich, die enantiomerenreine [(R)-1-Methylbutyl]malonsäure ausgehend von enantiomerenreinem (S)-2-Pentanol ohne Racemisierung herzustellen. Es ist auch erstmals möglich, [(R)-1-Methylbutyl]malonsäure durch Kristallisation von 95 % ee auf > 99 % ee anzureichern (analog Beispiel 8). Wegen des hohen erzielbaren Enantiomerenüberschusses von >95 % ee gelingt die Anreicherung durch Kristallisation technisch einfach und in sehr hoher Ausbeute. Enantiomerenreine (R)-1-Methylbutyl]malonsäure ist ein wertvolles organisches Zwischenprodukt.

Ein weiterer Gegenstand der Erfindung ist demnach (R)-1-Methylbutyl]malonsäure, die als Zwischenprodukt mittels des erfindungsgemäßen Verfahrens erhalten werden kann.

Die einfache Enantiomerenanreicherung auf der Stufe der Dicarbonsäuren der allgemeinen Formel (Va) oder (Vb) machen das erfindungsgemäße Verfahren auch aus diesem Grund besonders im technischen Maßstab interessant und wirtschaftlich.

Im Schritt D) erfolgt abschließend unter Erhalt der Konfiguration Decarboxylierung der nach Schritt C) erhaltenen Dicarbonsäuren, insbesondere solchen der allgemeinen Formel (Va) oder (Vb), zu den gewünschten Verfahrensprodukten (3-Alkylcarbonsäuren), insbesondere solchen der allgemeinen Formel (Ia) oder (Ib).

In einigen Fällen kann bereits unter Schritt C), insbesondere bei saurer Aufarbeitung oder erhöhten Temperaturen, ganz oder teilweise Decarboxylierung unter Bildung der 3-Alkylcarbonsäuren, insbesondere solchen der allgemeinen Formel (Ia) oder (Ib), eintreten. In diesen Fällen entfällt der ansonsten separat auszuführende Schritt D).

Die Decarboxylierung wird unter für den Fachmann bekannten Bedingungen durchgeführt. Die Decarboxylierung kann unter neutralen, sauren oder basischen Bedingungen erfolgen, bevorzugt unter neutralen und sauren Bedingungen.

Die Decarboxylierung erfolgt besonders bevorzugt in Form einer thermischen Decarboxylierung bei Temperaturen zwischen 40 und 250 °C, bevorzugt zwischen 80 und 180 °C. Zur thermischen Decarboxylierung wird die Dicarbonsäure, gegebenenfalls in Gegenwart eines Lösemittels, bis zur vollständigen Umsetzung erwärmt. Wird die Decarboxylierung in Gegenwart eines Lösemittels durchgeführt, dann kann das Lösemittel durch seinen Siedepunkt vorteilhaft den oberen Temperaturbereich begrenzen. Geeignete Lösemittel für die thermische Decarboxylierung werden insbesondere aus Toluol, Xylol oder Mesitylen ausgewählt.

Nach erfolgter Umsetzung wird das Lösemittel gegebenenfalls abdestilliert und das Produkt isoliert und gegebenenfalls durch Destillation, Kristallisation oder Chromatographie gereinigt.

Es ist auch möglich, durch Extraktion mit einem organischen Lösemittel im basischen Medium gegebenenfalls vorhandene organische Verunreinigungen von den 3-Alkylcarbonsäuren, insbesondere solchen der allgemeinen Formel (Ia) oder (Ib) bzw. deren korrespondierenden Salzen zu entfernen.

Es ist für kristalline Verfahrensprodukte, insbesondere solchen der allgemeinen Formel (Ia) oder (Ib), auch möglich, den Enantiomerenüberschuss durch Kristallisation bzw. Umkristallisation weiter zu erhöhen.

Der Druckbereich aller Teilschritte des erfindungsgemäßen Verfahrens ist unkritisch und kann innerhalb weiter Grenzen variiert werden. Der Druck beträgt üblicherweise 0.01 bis 20 bar, bevorzugt werden die Verfahrensschritte unter Normaldruck (Atmosphärendruck) durchgeführt.

Die Reaktion wird bevorzugt unter Inertisierung mit einem inerten Schutzgas, insbesondere Stickstoff oder Argon, durchgeführt.

Die Reaktion kann kontinuierlich oder diskontinuierlich, bevorzugt diskontinuierlich, durchgeführt werden.

Die Vorteile des erfindungsgemäßen Verfahrens liegen insbesondere darin, dass das Verfahren die Synthese der optisch aktiven 3-Alkylcarbonsäuren ausgehend von großtechnisch günstig verfügbaren enantiomerenreinen Alkoholen, die im Laufe der Reaktion einer vollständig stereoselektiven Inversion unterworfen werden, in gleichzeitig hohen chemischen und optischen Ausbeuten ermöglicht, ohne dass im Laufe der Synthese der aus dem Stand der Technik bekannte Verlust der stereochemischen Information des chiralen Zentrums durch Racemisierung oder partielle Racemisierung auftritt.

Ein besonderer Vorteil des erfindungsgemäßen Verfahrens ist, dass für den Schritt der stereospezifischen Substitution des aktivierten Alkohols mit Malonsäureester sehr hohe Ausbeuten von > 90 % bei gleichzeitig sehr hohen chemischen Reinheiten erzielt werden. Das Verfahren ermöglicht die Herstellung der gewünschten Produkte in sehr hohen Raum-Zeit-Ausbeuten, was das Verfahren für die industrielle Anwendung sehr attraktiv macht.

Das Verfahren stellt somit eine alternative Zugangsmöglichkeit zu 3-Alkylcarbonsäuren bereit.

Dadurch dass die Herstellung der optisch aktiven 3-Alkylcarbonsäuren unter Einsatz großtechnisch verfügbarer Edukte und Reagenzien mit sehr hohen chemischen und optischen Ausbeuten verläuft, gestaltet sich das Verfahren im Gegensatz zu den aus dem Stand der Technik bekannten Verfahren besonders wirtschaftlich und ist insbesondere für den großtechnischen Einsatz geeignet.

Die Durchführung des Verfahrens gestaltet sich insgesamt technisch einfach und sehr effizient. Es bedarf im Gegensatz zu den bekannten Verfahren auch keiner besonderen aufwändigen Verfahrensschritte, wie beispielsweise eine Tieftemperaturreaktion, oder des Einsatzes besonders teurer Hilfsstoffe, wie beispielsweise Butyllithium oder Cäsiumfluorid.

Zudem ermöglicht das Verfahren vorteilhaft auf der Stufe der Dicarbonsäuren, insbesondere solchen der allgemeinen Formel (Va) oder (Vb), eine technisch einfache und sehr effiziente Anreicherung des Enantiomerenüberschusses durch Kristallisation. Dadurch können vorteilhafterweise auch günstiger zugängliche sekundäre Alkohole als Edukte mit geringerer optischer Reinheit Verwendung finden. Dies macht das Verfahren besonders wirtschaftlich.

Alle vorstehenden Symbole der vorstehenden Formeln weisen ihre Bedeutungen jeweils unabhängig voneinander auf.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C. Die Beispiele dienen der weiteren Veranschaulichung des erfindungsgemäßen Verfahrens und sind in keiner Weise als Einschränkung zu betrachten.

### Beispiel 1:

### Herstellung von (R)-3-Methylheptansäure

### 1a: Herstellung von (S)-Methansulfonsäure-(1-methylpentyl)ester

Bei Raumtemperatur wurden in einem Dreihalskolben mit Innenthermometer, Tropftrichter und Rührer unter Stickstoff-Schutzgas 54.9 g (S)-2-Hexanol (> 99 % ee, 0.537 mol) und 70.7 g Triethylamin (0.7 mol) in 550 ml Methylenchlorid vorgelegt. Zu der Mischung wurden bei 0 °C 80 g Methansulfonsäurechlorid (0.7 mol) innerhalb 30 min unverdünnt zugefügt, wobei sich ein Niederschlag bildete. Anschließend rührte man die Mischung 60 min bei 0 °C, hydrolysierte mit 300 ml gesättigter Natriumhydrogencarbonat-Lösung und rührte die Mischung 15 min. Nach Phasentrennung wurde mit 300 ml Wasser gewaschen und die organische Phase dann über Natriumsulfat getrocknet. Nach Abdestillieren von Lösemittel wurde (S)-Methansulfonsäure-(1-methylpentyl)ester in einer Ausbeute von 96.6 g (99 % d. Th.) und einer chemischen Reinheit von 98 % (GC) erhalten.

### 1b: Herstellung von [(R)-1-Methylpentyl]malonsäurediethylester

Bei Raumtemperatur wurden in einem Dreihalskolben mit Innenthermometer, Tropftrichter und Rührer unter Stickstoff-Schutzgas 14.7 g Natriumhydrid (95 %-ig, 0.583 mol) in 400 ml trockenem 1,2-Dimethoxyethan vorgelegt. Anschließend wurden bei 25 bis 30 °C unter Wasserstoffentwicklung 97.5 g Diethylmalonat (0.609 mol) zugetropft. Man rührte die klare Mischung 30 min bei 25 °C und fügte 93 g (S)-Methansulfonsäure-(1-methylpentyl)ester (0.516 mol) unverdünnt zu. Die Mischung wurde auf 85 °C erwärmt und 5 h gerührt. Nach Abkühlung auf 25 °C wurde die Suspension mit 600 ml Ether und 500 ml gesättigter Ammoniumchlorid-Lösung versetzt. Nach Phasentrennung wurde die organische Phase mit 250 ml Wasser gewaschen und dann über Natriumsulfat getrocknet. Nach Abdestillieren von Lösemittel wurde [(R)-1-Methylpentyl]malonsäurediethylester in einer Ausbeute von 123 g (98 % d. Th.) und einer chemischen Reinheit von 96 % (GC) erhalten.

### 1c: Herstellung von [(R)-1-Methylpentyl]malonsäure

Bei Raumtemperatur wurden in einem Dreihalskolben mit Innenthermometer, Tropftrichter und Rührer 81 g Natriumhydroxid (2 mol) in 360 ml Wasser und 160 ml Methanol gelöst. Dann wurden 124 g [(R)-1-Methylpentyl]malonsäurediethylester (0.51 mol) zugegeben. Man erwärmte die Mischung auf 70 °C und rührte 3.5 h bei dieser Temperatur, wobei sich ein Feststoff bildete. Nach Abkühlung auf 25 °C wurde die Suspension mit 250 ml Ether und unter Kühlung mit 400 ml 20 Gew. %-iger Salzsäure versetzt. Nach Phasentrennung wurde die wässrige Phase zweimal mit 100 ml Ether extrahiert. Die vereinten organischen Phasen wurden mit 100 ml Wasser gewaschen und dann über Natriumsulfat getrocknet. Nach Abdestillieren von Lösemittel wurde [(R)-1-Methylpentyl]malonsäure in einer Ausbeute von 85 g (89 % d. Th.) erhalten (Smp.: 123 °C).

### 1d: Herstellung von (R)-3-Methylheptansäure

Bei Raumtemperatur wurden in einem Dreihalskolben mit Innenthermometer, Kühler und Rührer 60 g [(R)-1-Methylpentyl]malonsäure (0.318 mol) in 120 ml Xylol gelöst. Man erwärmte die Mischung 5 h auf 125 °C. Nach Abkühlung auf 25 °C wurde mit 200 ml 2 N Natronlauge versetzt. Nach Phasentrennung wurde die wässrige Phase mit 200 ml Ether und unter Kühlung mit 180 ml 15 Gew. %-iger Salzsäure versetzt. Nach Phasentrennung wurde die wässrige Phase zweimal mit 100 ml Ether extrahiert. Die vereinten organischen Phasen wurden mit 50 ml Wasser gewaschen und dann über Natriumsulfat getrocknet. Nach Abdestillieren von Lösemittel wurde (R)-3-Methylheptansäure destilliert und in einer Ausbeute von 35.5 g (77 % d. Th.) mit > 99 % ee und > 99 % chemischer Reinheit erhalten (Sdp.: 67-68 °C bei 0.45 mbar).

Die gesamte Synthesesequenz 1 a bis 1 d verläuft ohne Racemisierung.

### Beispiel 2:

### Herstellung von (R)-3-Methylheptansäure

Analog zu Beispiel 1 wurde (R)-3-Methylheptansäure in 65 % d. Th. über alle vier Stufen ausgehend von (S)-2-Hexanol (> 99 % ee), das 20 Mol-% 2-Hexanon als korrespondierendes prochirales Keton enthielt (Edukt für die biokatalytische Reduktion), erhalten. 2-Hexanon ließ sich auf der Stufe des (S)-Methansulfonsäure-(1-methylpentyl)ester problemlos durch Destillation entfernen.

### Beispiel 3:

### Herstellung von (R)-3-Methylheptansäure

Analog zu Beispiel 1 wurde (R)-3-Methylheptansäure in 67 und 69 % d. Th. über alle vier Stufen erhalten, wenn die Herstellung - von [(R)-1-Methylpentyl]malonsäurediethylester in Diethylenglykol-dimethylether und 1,2-Diethoxyethan als Lösemittel durchgeführt wurde.

### Beispiel 4:

### Herstellung von (R)-3-Methylhexansäure

### 4a: Herstellung von (S)-Methansulfonsäure-(1-methylbutyl)ester

Analog Beispiel 1 a wurde bei Verwendung von 22.8 g (0.26 mol) (S)-2-Pentanol (98.4 % ee) (S)-Methansulfonsäure-(1-methylbutyl)ester in einer Ausbeute von 42.1 g (98 % d. Th.) und einer chemischen Reinheit von 97 % (GC) erhalten.

### 4b: Herstellung von [(R)-1-Methylbutyl]malonsäurediethylester

Analog Beispiel 1 b wurde bei Verwendung von 42.1 g (0.253 mol) (S)-Methansulfonsäure-(1-methylbutyl)ester [(R)-1-Methylbutyl]malonsäurediethylester in einer Ausbeute von 56.5 g (97 % d. Th.) und einer chemischen Reinheit von 96 % (GC) erhalten.

### 4c: Herstellung von [(R)-1-Methylbutyl]malonsäure

Analog Beispiel 1 c wurde bei Verwendung von 56.5 g (0.245 mol) [(R)-1-Methylbutyl]malonsäurediethylester [(R)-1-Methylbutyl]malonsäure in einer Ausbeute von 40.5 g (95 % d. Th.) erhalten (Smp.: 95 °C).

### 4d: Herstellung von (R)-3-Methylhexansäure

Analog Beispiel 1 d wurde bei Verwendung von 40.5 g (0.232 mol) [(R)-1-Methylbutyl]malonsäure (R)-3-Methylhexansäure in einer Ausbeute von 25.5 g (84 % d. Th.) mit 98.4 % ee und > 99 % chemischer Reinheit erhalten (Sdp.: 81 °C bei 2 mbar).

Die gesamte Synthesesequenz 4 a bis 4 d verläuft ohne Racemisierung.

### Beispiel 5:

### Herstellung von (R)-3-Ethylheptansäure

Analog Beispiel 1 wurde ausgehend von (S)-3-Heptanol (> 99 % ee) (R)-3-Ethylheptansäure in einer Ausbeute von 66 % d. Th. über alle vier Stufen mit > 99 % ee und > 99 % chemischer Reinheit erhalten.

### Beispiel 6:

### Herstellung von (S)-5-Benzyloxy-3-ethylpentansäure

Analog Beispiel 1 wurde ausgehend von (R)-1-Benzyloxypentan-3-ol (> 99 % ee) (S)-5-Benzyloxy-3-ethylpentansäure in einer Ausbeute von 61 % d. Th. über alle vier Stufen mit > 99 % ee und > 99 % chemischer Reinheit erhalten.

### Beispiel 7:

### Herstellung von (R)-3-Methyl-5-phenylpentansäure

Analog Beispiel 1 wurde ausgehend von (S)-4-Phenylbutan-2-ol (> 99 % ee) (R)-3-Methyl-5-phenylpentansäure in einer Ausbeute von 63 % d. Th. über alle vier Stufen mit > 99 % ee und > 99 % chemischer Reinheit erhalten.

### Beispiel 8:

### Umkristallisation und Anreicherung von [(R)-1-Methylpentyl]malonsäure

24.8 g [(R)-1-Methylpentyl]malonsäure mit 95 % ee, hergestellt analog Beispiel 1 ausgehend von 2-Hexanol mit 95 % ee, wurden in 120 ml n-Hexan am Rückfluss gelöst. Die klare Lösung wurde innerhalb 6 h auf 18 °C abgekühlt. Der gebildete Niederschlag wurde abfiltriert und mit 50 ml kaltem n-Hexan gewaschen und im Vakuum getrocknet. Es wurden 22 g [(R)-1-Methylpentyl]malonsäure in einer Ausbeute von 89 % d. Th. mit > 99 % ee erhalten.

### Beispiel 9:

### Herstellung von (R)-3-Methylheptansäure

### 9a: Herstellung von (S)-Methansulfonsäure-(1-methylpentyl)ester

Die Herstellung erfolgt analog Beispiel 1 a.

### 9b: Herstellung von [(R)-1-Methylpentyl]malonsäurediethylester

Bei Raumtemperatur wurden in einem Dreihalskolben mit Innenthermometer, Tropftrichter und Rührer unter Stickstoff-Schutzgas 14.7 g Natriumhydrid (95 %-ig, 0.583 mol) in 290 ml trockenem 1,2-Dimethoxyethan und 50 ml trockenem Dimethylformamid vorgelegt. Anschließend wurden bei 25 bis 30 °C unter Wasserstoffentwicklung 97.5 g Diethylmalonat (0.609 mol) zugetropft. Man rührte die klare Mischung 30 min bei 25 °C und fügte 93 g (S)-Methansulfonsäure-(1-methylpentyl)ester (0.516 mol) unverdünnt zu. Die Mischung wurde auf 80 °C erwärmt und 5 h gerührt. Nach Abkühlung auf 25 °C wurde die Suspension mit 600 ml Ether und 500 ml gesättigter Ammoniumchlorid-Lösung versetzt. Nach Phasentrennung wurde die organische Phase mit 250 ml Wasser gewaschen und dann über Natriumsulfat getrocknet. Nach Abdestillieren von Lösemittel wurde [(R)-1-Methylpentyl]malonsäurediethylester in einer Ausbeute von 119 g (94 % d. Th.) und einer chemischen Reinheit von 95 % (GC) erhalten.

### 9c: Herstellung von [(R)-1-Methylpentyl]malonsäure

Die Herstellung erfolgt analog Beispiel 1c.

### 9d: Herstellung von (R)-3-Methylheptansäure

analog Beispiel 1 d. (R)-3-Methylheptansäure wurde in einer Ausbeute von 64 % d. Th. über alle Stufen mit > 99 % ee und > 99 % chemischer Reinheit erhalten. Racemisierung im Schritt 9 b wurde nicht beobachtet.

### Beispiel 10:

### Herstellung von (R)-3-Methylhexansäure

### 10a: Herstellung von (S)-Methansulfonsäure-(1-methylbutyl)ester

Die Herstellung erfolgt analog Beispiel 1 a.

### 10b: Herstellung von [(R)-1-Methylbutyl]malonsäurediethylester

Bei Raumtemperatur wurden in einem Dreihalskolben mit Innenthermometer, Tropftrichter und Rührer unter Stickstoff-Schutzgas 41.8 g Natriumethanolat (95 %-ig, 0.583 mol) in 250 ml trockenem 1,2-Dimethoxyethan und 30 ml trockenem Ethanol vorgelegt. Anschließend wurden bei 20 bis 40 °C 97.5 g Diethylmalonat (0.609 mol) zugetropft, wobei sich eine klare Lösung bildete. Man rührte die klare Mischung 30 min bei 40 °C und fügte 85.7 g (S)-Methansulfonsäure-(1-methylbutyl)ester (0.516 mol) unverdünnt zu. Die Mischung wurde auf 80 °C erwärmt und 6 h gerührt. Nach Abkühlung auf 25 °C wurde die Suspension mit 800 ml Methylenchlorid und 500 ml 5 Gew.-%-iger Ammoniumchlorid-Lösung versetzt. Nach Phasentrennung wurde die organische Phase mit 200 ml Wasser gewaschen und dann über Natriumsulfat getrocknet. Nach Abdestillieren von Lösemittel wurde [(R)-1-Methylbutyl]malonsäurediethylester in einer Ausbeute von 113 g (95 % d. Th.) und einer chemischen Reinheit von 96 % (GC) erhalten.

### 10c: Herstellung von [(R)-1-Methylbutyl]malonsäure

Die Herstellung erfolgt analog Beispiel 1 c.

### 10d: Herstellung von (R)-3-Methylhexansäure

analog Beispiel 1 d. (R)-3-Methylhexansäure wurde in einer Ausbeute von 62 % d. Th. über alle Stufen mit > 99 % ee und > 99 % chemischer Reinheit erhalten. Racemisierung im Schritt 10 b wurde nicht beobachtet.

### Beispiel 11:

### Herstellung von (R)-3-Methylhexansäure

### 11a: Herstellung von (S)-Methansulfonsäure-(1-methylbutyl)ester

Die Herstellung erfolgt analog Beispiel 1 a.

### 11b: Herstellung von [(R)-1-Methylbutyl]malonsäurediethylester

Bei Raumtemperatur wurden in einem Dreihalskolben mit Innenthermometer, Tropftrichter und Rührer unter Stickstoff-Schutzgas 33.1 g Natriummethoxid (95 %-ig, 0.583 mol) in 250 ml trockenem Essigsäureethylester vorgelegt. Anschließend wurden bei 20 bis 30 °C 97.5 g Diethylmalonat (0.609 mol) zugetropft. Man rührte die klare Mischung 30 min bei 25 °C und fügte 85.7 g (S)-Methansulfonsäure-(1-methylbutyl)ester (0.516 mol) unverdünnt zu. Die Mischung wurde auf 79 °C erwärmt und 7 h gerührt. Nach Abkühlung auf 25 °C wurde die Suspension mit 800 ml Methylenchlorid und 500 ml 5 Gew.-%-iger Ammoniumchlorid-Lösung versetzt. Nach Phasentrennung wurde die organische Phase mit 150 ml Wasser gewaschen und dann über Natriumsulfat getrocknet. Nach Abdestillieren von Lösemittel wurde [(R)-1-Methylbutyl]malonsäurediethylester in einer Ausbeute von 105 g erhalten. Das Produkt enthält Dimethyl- und Ethylmethylester, gebildet durch Umesterung (GC-Reinheit: Summe aus Me/Me, Me/Et, Et/Et: 96 %). Die korrigierte Ausbeute beträgt 96 % d.Th.

### 11c: Herstellung von [(R)-1-Methylbutyl]malonsäure

Die Herstellung erfolgt analog Beispiel 1 c.

### 11d: Herstellung von (R)-3-Methylhexansäure

analog Beispiel 1 d. (R)-3-Methylhexansäure wurde in einer Ausbeute von 62 % d. Th. über alle Stufen mit 98.8 % ee und > 99 % chemischer Reinheit erhalten (die optische Reinheit ist von > 99 % ee auf 98.8 % ee gesunken).

### Vergleichsbeispiel 1:

### Herstellung von (R)-3-Methylheptansäure (Alkylierung von Malonat in Dimethylformamid)

### Vla: Herstellung von (S)-Methansulfonsäure-(1-methylpentyl)ester

Die Herstellung erfolgt analog Beispiel 1 a ((S)-2-Hexanol: >99.9 % ee).

### V1b: Herstellung von [(R)-1-Methylpentyl]malonsäurediethylester

Bei Raumtemperatur wurden in einem Dreihalskolben mit Innenthermometer, Tropftrichter und Rührer unter Stickstoff-Schutzgas 14.7 g Natriumhydrid (95 %-ig, 0.583 mol) in 300 ml trockenem Dimethylformamid vorgelegt. Anschließend wurden bei 25 bis 30 °C unter Wasserstoffentwicklung 97.5 g Diethylmalonat (0.609 mol) zugetropft. Man rührte die klare Mischung 30 min bei 25 °C und fügte 93 g (S)-Methansulfonsäure-(1-methylpentyl)ester (0.516 mol) unverdünnt zu. Die Mischung wurde auf 85 °C erwärmt und 5 h gerührt. Nach Abkühlung auf 25 °C wurde die Suspension mit 1000 ml Methylenchlorid und 600 ml 5 Gew.-%-iger Ammoniumchlorid-Lösung versetzt. Nach Phasentrennung wurde die organische Phase mit 200 ml Wasser gewaschen und dann über Natriumsulfat getrocknet. Nach Abdestillieren von Lösemittel wurde [(R)-1-Methylpentyl]malonsäurediethylester destilliert (85 °C, 0.6 mbar) und in einer Ausbeute von 48 g (38 % d. Th.) erhalten.

### V1c: Herstellung von [(R)-1-Methylpentyl]malonsäure

Die Herstellung erfolgt analog Beispiel 1 c.

### V1d: Herstellung von (R)-3-Methylheptansäure

analog Beispiel 1 d. (R)-3-Methylheptansäure wurde in einer Ausbeute von 26 % d. Th. über alle Stufen mit 96.3 % ee und 97 % chemischer Reinheit erhalten (Racemisierung).

### Vergleichsbeispiel 2:

### Herstellung von (R)-3-Methylheptansäure (Alkylierung von Malonat in Alkohol)

### V2a: Herstellung von (S)-Methansulfonsäure-(1-methylpentyl)ester

Die Herstellung erfolgt analog Beispiel 1 a ((S)-2-Hexanol: > 99.9 % ee).

### V2b: Herstellung von [(R)-1-Methylpentyl]malonsäuredimethylester

Bei Raumtemperatur wurden in einem Dreihalskolben mit Innenthermometer, Tropftrichter und Rührer unter Stickstoff-Schutzgas 28.9 g Natriummethanolat (95 %-ig, 0.51 mol) in 150 ml trockenem Methanol gelöst. Anschließend wurden bei 30 bis 35 °C 85.3 g Diethylmalonat (0.533 mol) zugetropft, wobei sich zunächst eine Suspension bildete, die sich gegen Ende der Zugabe auflöste. Man rührte die klare Mischung 60 min bei 50 °C und fügte 80 g (S)-Methansulfonsäure-(1-methylpentyl)ester (0.444 mol) unverdünnt zu. Die Mischung wurde auf 66 °C erwärmt und 6 h gerührt. Nach Abkühlung auf 25 °C wurde die Suspension mit 400 ml Methylenchlorid und 300 ml 5 Gew.-%-iger Ammoniumchlorid-Lösung versetzt. Nach Phasentrennung wurde die organische Phase mit 100 ml Wasser gewaschen und dann über Natriumsulfat getrocknet. Nach Abdestillieren von Lösemittel wurde [(R)-1-Methylpentyl]malonsäurediethylester in einer Ausbeute von 94 g erhalten. Das Produkt enthält Dimethyl- und Ethylmethylester, gebildet durch Umesterung, sowie nichtumgesetzten Malonester. Die korrigierte Ausbeute beträgt 84 % d.Th.

### V2c: Herstellung von [(R)-1-Methylpentyl]malonsäure

Die Herstellung erfolgt analog Beispiel 1 c.

### V2d: Herstellung von (R)-3-Methylheptansäure

analog Beispiel 1 d. (R)-3-Methylheptansäure wurde in einer Ausbeute von 56 % d. Th. über alle Stufen mit 97.3 % ee und 97 % chemischer Reinheit erhalten (Racemisierung).

Analoge Durchführung bei Verwendung von Ethanol und Isopropanol als Lösemittel für die Alkylierung von Malonat in V2b liefert (R)-3-Methylheptansäure mit 97.4 und 98.0 % ee (Racemisierung).

## Patentansprüche

1. Verfahren zur Herstellung optisch aktiver 3-Alkylcarbonsäuren enthaltend die folgenden Schritte:
A) Überführung eines optisch aktiven sekundären Alkohols in eine optisch aktive, aktivierte Verbindung durch Einführung einer Abgangsgruppe
B) Umsetzung der aktivierten Verbindung mit einem Malonsäurederivat zu einer optisch aktiven, alkylierten Malonsäureverbindung, wobei die Umsetzung ausschließlich in Gegenwart eines oder mehrerer Lösemittel ausgewählt aus der Klasse der Ether oder der Carbonsäureester erfolgt, wobei optional ein Zusatz an einem oder mehreren aprotisch polaren Lösemitteln oder Alkoholen als Cosolvens erfolgen kann, wobei dieser Zusatz maximal 30% des insgesamt zugesetzten Lösemittelvolumens beträgt mit der Maßgabe, dass es sich bei dem zugesetzten Cosolvens nicht um Hexamethylphosphorsäuretriamid handelt
C) gegebenenfalls Verseifung der Malonsäureverbindung zur korrespondierenden Säure und
D) abschließende Decarboxylierung der korrespondierenden Säure.

2. Verfahren nach Anspruch 1 zur Herstellung optisch aktiver 3-Alkylcarbonsäuren der allgemeinen Formel (Ia) oder (Ib), 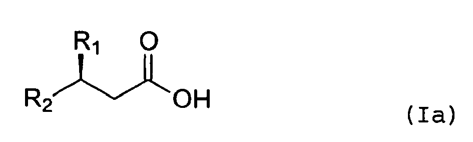 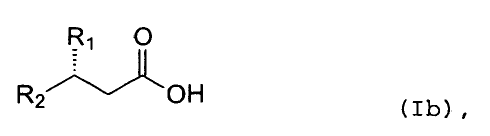 wobei **R¹** und **R²** unabhängig voneinander für einen linearen oder verzweigten, gesättigten oder ungesättigten, cyclischen oder cyclische Gruppen enthaltenden gegebenenfalls mit Q substituierten C₁-C₁₅-Kohlenwasserstoff-Rest stehen
und Q ausgewählt wird aus der Gruppe enthaltend Halogen, Amino, Amido, Phthalimido, Hydroxy, Cyano, Nitro, Alkoxy, Aryloxy, Aralkyloxy, Alkylthio, Acyl, Silyl, Silyloxy, Aryl, Heteroaryl,
enthaltend die folgenden Schritte:
A) Umsetzung einer Verbindung der allgemeinen Formel (IIa) oder (IIb) 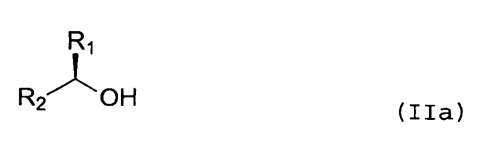 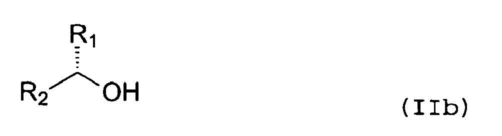 zu aktivierten Verbindungen der allgemeinen Formel (IIIa) oder (IIIb) 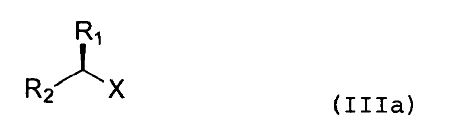 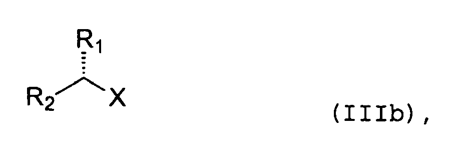 wobei
X ausgewählt wird aus der Gruppe enthaltend Halogen, OSO₂R, OSO₂OR oder OCOR und
R für einen linearen oder verzweigten, gesättigten oder ungesättigten, cyclischen oder cyclische Gruppen enthaltenden gegebenenfalls mit Q substituierten C₁-C₁₅-KohlenwasserstoffRest oder Fluor steht
B) Umsetzung der nach Schritt A) erhaltenen Verbindungen der allgemeinen Formel (IIIa) oder (IIIb) mit einem Malonsäurederivat unter Erhalt von Verbindungen der allgemeinen Formel (IVa) oder (IVb), wobei die Umsetzung ausschließlich in Gegenwart eines oder mehrerer Lösemittel ausgewählt aus der Klasse der Ether oder der Carbonsäureester erfolgt, wobei optional ein Zusatz an einem oder mehreren aprotisch polaren Lösemitteln oder Alkoholen als Cosolvens erfolgen kann, wobei dieser Zusatz maximal 30% des insgesamt zugesetzten Lösemittelvolumens beträgt mit der Maßgabe, dass es sich bei dem zugesetzten Cosolvens nicht um Hexamethylphosphorsäuretriamid handelt und 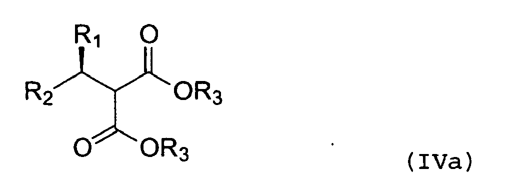 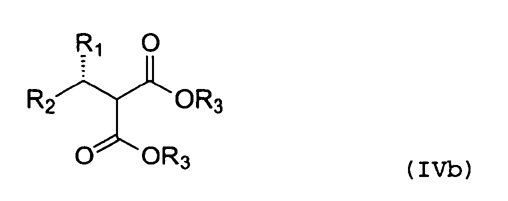 wobei
**R³** in der allgemeinen Formel (IVa) oder (IVb) jeweils unabhängig voneinander für Wasserstoff oder einen linearen oder verzweigten, gesättigten oder ungesättigten, cyclischen oder cyclische Gruppen enthaltenden C₁-C₁₅-Kohlenwasserstoff-Rest, einen C₁-C₂₀-Trialkyl- oder C₁-C₂₀-Triaryl-Silyl-Rest steht,
C) gegebenenfalls Verseifung der nach Schritt B) erhaltenen Verbindungen der allgemeinen Formel (IVa) oder (IVb) unter Erhalt von Dicarbonsäuren der allgemeinen Formel (Va) oder (Vb) 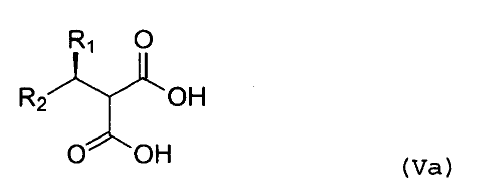 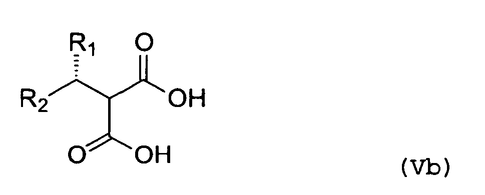
D) und abschließende Decarboxylierung der nach Schritt C) erhaltenen Dicarbonsäure der allgemeinen Formel (Va) oder (Vb).

3. Verfahren nach Anspruch 1 oder 2, **dadurch gekennzeichnet, dass** in der Umsetzung nach Schritt B) bei einem optionalen Zusatz eines oder mehrerer polar aprotischer Lösemittel als Cosolventien diese ausgewählt werden aus Klasse der Lösemittel enthaltend Nitrile, Carbonsäureamide, Harnstoffderivate und Sulfoxide.

4. Verfahren nach Anspruch 1 oder 2, **dadurch** gekennzeichent, dass in der Umsetzung nach Schritt B) bei einem optionalen Zusatz eines oder mehrerer polar aprotischer Lösemittel als Cosolventien diese ausgewählt werden aus der Gruppe enthaltend Acetonitril, Propionitril, Butyronitril, Formamid, N-Methylformamid, Dimethylformamid, Diethylformamid, Dimethylacetamid, Diethylacetamid, N-Methylpyrrolidon, 1,3-Dimethyl-3,4,5,6-tetrahydro-2(1H)-pyrimidinon und Dimethylsulfoxid.

5. Verfahren nach Anspruch 1 oder 2, **dadurch** gekennzeichent, dass bei der Umsetzung nach Schritt B) bei einem optionalen Zusatz an Alkohol als Cosolvens dieser ausgewählt wird aus der Gruppe enthaltend Methanol, Ethanol, Propanol, Isopropanol, Butanol, Isobutanol, tert.-Butanol, Pentanol und Amylalkohol.

6. Verfahren nach einem oder mehreren der Ansprüche 1 bis 5, **dadurch gekennzeichnet, dass** der Zusatz an an einem oder mehreren aprotisch polaren Lösemitteln oder Alkoholen als Cosolvens maximal 20 % des insgesamt zugesetzten Lösemittelvolumens beträgt.

7. Verfahren nach Anspruch 1 oder 2, **dadurch gekennzeichnet, dass** die Umsetzung nach Schritt B) ausschließlich in Gegenwart eines oder mehrerer Lösemittel aus der Klasse der Ether oder der Carbonsäureester durchgeführt wird.

8. Verfahren nach Anspruch 1 oder 2, **dadurch gekennzeichnet, dass** die Umsetzung nach Schritt B) ausschließlich in Gegenwart eines oder mehrerer Lösemittel aus der Klasse der Ether durchgeführt wird.

9. Verfahren nach einem oder mehreren der Ansprüche 1 bis 8, **dadurch gekennzeichnet, dass** die nach Schritt C) erhaltenen Verbindungen in einem zusätzlichen Schritt vor der Decarboxylierung gereinigt werden.

10. Verfahren nach einem oder mehreren der Ansprüche 1 bis 9, **dadurch gekennzeichnet, dass** Schritt B) in Gegenwart eines oder mehrerer Lösemittel ausgewählt aus der Gruppe enthaltend Tetrahydrofuran, 2-Methyltetrahydrofuran, 2,5-Dimethyltetrahydrofuran, 2,5-Dimethoxytetrahydrofuran, 1,4-Dioxan, Dimethoxyethan, Diethoxyethan, Diethylenglykol-dimethylether, Diethylenglykol-diethylether, Diethylenglykol-dipropylether, Diethylenglykol-dibutylether, Triethylenglykol-dimethylether, Triethylenglykol-diethylether und Triethylenglykol-dibutylether durchgeführt wird.

11. Verfahren nach einem oder mehreren der Ansprüche 1 bis 9, **dadurch gekennzeichnet, dass** Schritt B) ausschließlich in Gegenwart eines oder mehrerer Lösemittel ausgewählt aus der Gruppe enthaltend Tetrahydrofuran, 2-Methyltetrahydrofuran, 2,5-Dimethyltetrahydrofuran, 2,5-Dimethoxytetrahydrofuran, 1,4-Dioxan, Dimethoxyethan, Diethoxyethan, Diethylenglykol-dimethylether, Diethylenglykol-diethylether, Diethylenglykol-dipropylether, Diethylenglykol-dibutylether, Triethylenglykol-dimethylether, Triethylenglykol-diethylether und Triethylenglykol-dibutylether und Essigsäureethylester, Essigsäurepropylester, Essigsäureisopropylester und Essigsäurebutylester durchgeführt wird.

12. Verfahren nach einem oder mehreren der Ansprüche 1 bis 11 **dadurch gekennzeichnet, dass** zur Enantiomerenanreicherung die Verbindungen der allgemeinen Formel (Va) oder (Vb) in einem zusätzlichen Schritt kristallisiert oder umkristallisiert werden.

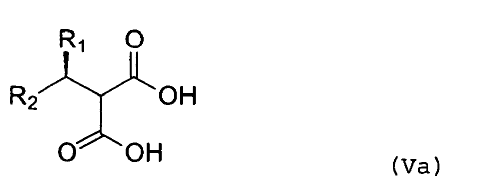

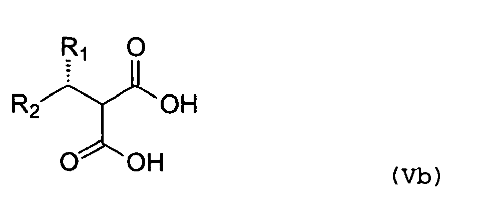

## Claims

1. Process for preparing optically active 3-alkylcarboxylic acids, comprising the following steps:
A) conversion of an optically active secondary alcohol to an optically active, activated compound by introducing a leaving group,
B) reacting the activated compound with a malonic acid derivative to give an optically active, alkylated malonic acid compound, the reaction being effected exclusively in the presence of one or more solvents selected from the class of the ethers or of the carboxylic esters, one or more aprotic polar solvents or alcohols optionally being added as a cosolvent, this addition being a maximum of 30% of the total volume of solvent added, with the proviso that the cosolvent added is not hexamethylphosphoramide,
C) optionally hydrolyzing the malonic acid compound to the corresponding acid and
D) finally decarboxylating the corresponding acid.

2. Process according to Claim 1 for preparing optically active 3-alkylcarboxylic acids of the general formula (Ia) or (Ib) 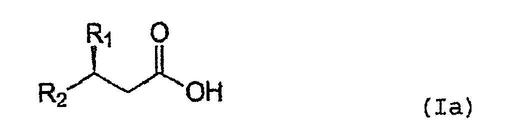 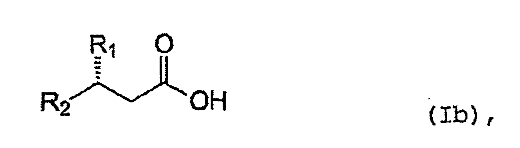 where **R¹** and **R²** are each independently a linear or branched, saturated or unsaturated C₁-C₁₅ hydrocarbon radical which is cyclic or contains cyclic groups and is optionally substituted by Q
and Q is selected from the group comprising halogen, amino, amido, phthalimido, hydroxyl, cyano, nitro, alkoxy, aryloxy, aralkyloxy, alkylthio, acyl, silyl, silyloxy, aryl, heteroaryl,
comprising the following steps:
A) converting a compound of the general formula (IIa) or (IIb) 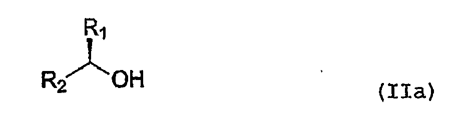 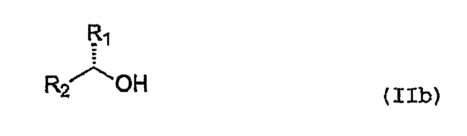 to activated compounds of the general formula (IIIa) or (IIIb) 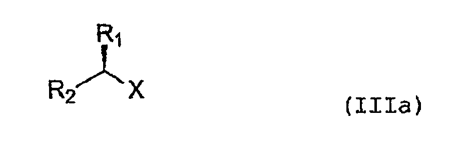 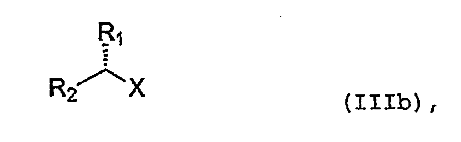 where
**X** is selected from the group comprising halogen, OSO₂R, OSO₂OR or OCOR and
**R** is a linear or branched, saturated or unsaturated C₁-C₁₅ hydrocarbon radical which is cyclic or contains cyclic groups and is optionally substituted by Q, or is fluorine,
B) reacting the compounds of the general formula (IIIa) or (IIIb) obtained in step A) with a malonic acid derivative to obtain compounds of the general formula (IVa) or (IVb), the reaction being effected exclusively in the presence of one or more solvents from the class of the ethers or of the carboxylic esters, one or more aprotic polar solvents or alcohols optionally being added as a cosolvent, this addition being a maximum of 30% of the total volume of solvent added, with the proviso that the cosolvent added is not hexamethylphosphoramide, and 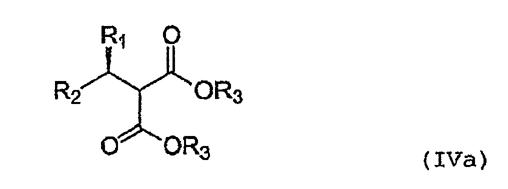 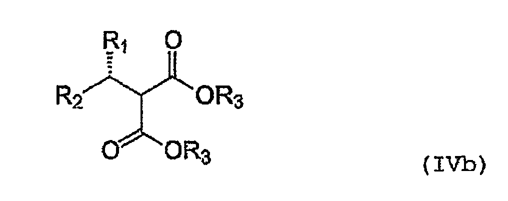 where
R³ in the general formula (IVa) or (IVb) is in each case independently hydrogen or a linear or branched, saturated or unsaturated C₁-C₁₅ hydrocarbon radical which is cyclic or contains cyclic groups, or is a C₁-C₂₀ trialkyl- or C₁-C₂₀ triarylsilyl radical,
C) optionally hydrolyzing the compounds of the general formula (IVa) or (IVb) obtained in step B) to obtain dicarboxylic acids of the general formula (Va) or (Vb) 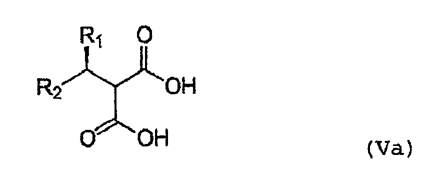 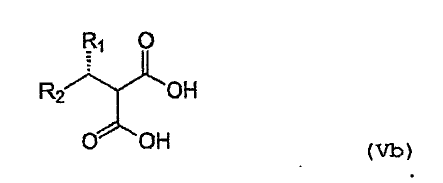
D) and finally decarboxylating the dicarboxylic acid of the general formula (Va) or (Vb) obtained in step C).

3. Process according to Claim 1 or 2, **characterized in that**, in the reaction in step B), in the case of optional addition of one or more polar aprotic solvents as cosolvents, they are selected from the class of the solvents comprising nitriles, carboxamides, urea derivatives and sulfoxides.

4. Process according to Claim 1 or 2, **characterized in that**, in the reaction in step B), in the case of optional addition of one or more polar aprotic solvents as cosolvents, they are selected from the group comprising acetonitrile, propionitrile, butyronitrile, formamide, N-methylformamide, dimethylformamide, diethylformamide, dimethylacetamide, diethylacetamide, N-methylpyrrolidone, 1,3-dimethyl-3,4,5,6-tetrahydro-2(1H)-pyrimidinone and dimethyl sulfoxide.

5. Process according to Claim 1 or 2, **characterized in that**, in the reaction in step B), in the case of optional addition of alcohol as a cosolvent, the alcohol is selected from the group comprising methanol, ethanol, propanol, isopropanol, butanol, isobutanol, tert-butanol, pentanol and amyl alcohol.

6. Process according to one or more of Claims 1 to 5, **characterized in that** the addition of one or more aprotic polar solvents or alcohols as a cosolvent is a maximum of 20% of the total volume of solvent added.

7. Process according to Claim 1 or 2, **characterized in that** the reaction in step B) is performed exclusively in the presence of one or more solvents from the class of the ethers or of the carboxylic esters.

8. Process according to Claim 1 or 2, **characterized in that** the reaction in step B) is performed exclusively in the presence of one or more solvents from the class of the ethers.

9. Process according to one or more of Claims 1 to 8, **characterized in that** the compounds obtained in step C) are purified in an additional step before the decarboxylation.

10. Process according to one or more of Claims 1 to 9, **characterized in that** step B) is performed in the presence of one or more solvents selected from the group comprising tetrahydrofuran, 2-methyltetrahydrofuran, 2,5-dimethyltetrahydrofuran, 2,5-dimethoxytetrahydrofuran, 1,4-dioxane, dimethoxyethane, diethoxyethane, diethylene glycol dimethyl ether, diethylene glycol diethyl ether, diethylene glycol dipropyl ether, diethylene glycol dibutyl ether, triethylene glycol dimethyl ether, triethylene glycol diethyl ether and triethylene glycol dibutyl ether.

11. Process according to one or more of Claims 1 to 9, **characterized in that** step B) is performed exclusively in the presence of one or more solvents selected from the group comprising tetrahydrofuran, 2-methyltetrahydrofuran, 2,5-dimethyltetrahydrofuran, 2,5-dimethoxytetrahydrofuran, 1,4-dioxane, dimethoxyethane, diethoxyethane, diethylene glycol dimethyl ether, diethylene glycol diethyl ether, diethylene glycol dipropyl ether, diethylene glycol dibutyl ether, triethylene glycol dimethyl ether, triethylene glycol diethyl ether and triethylene glycol dibutyl ether and ethyl acetate, propyl acetate, isopropyl acetate and butyl acetate.

12. Process according to one or more of Claims 1 to 11, **characterized in that** the compounds of the general formula (Va) or (Vb) are enantiomerically enriched by crystallization or recrystallization in an additional step.

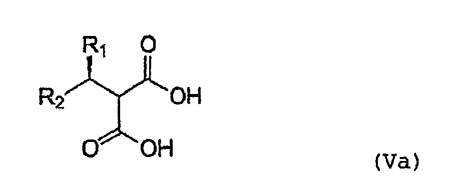

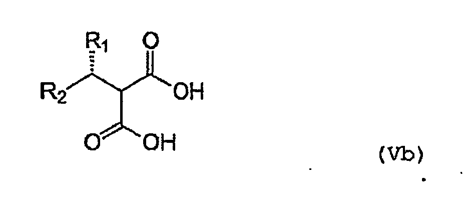

## Revendications

1. Procédé pour la préparation d'acides 3-alkylcarboxyliques optiquement actifs, comportant les étapes suivantes:
(A) conversion d'un alcool secondaire optiquement actif en un composé activé, optiquement actif, par introduction d'un groupe partant
(B) mise en réaction du composé activé avec un dérivé d'acide malonique, pour l'obtention d'un composé acide malonique alkylé optiquement actif, la réaction s'effectuant exclusivement en présence d'un ou plusieurs solvant choisi(s) dans la classe des éthers ou des esters d'acides carboxyliques, une addition d'un ou plusieurs solvants polaires aprotiques ou alcools en tant que co-solvant(s) pouvant éventuellement être effectuée, cette addition représentant au maximum 30 % du volume de solvants ajoutés au total, étant entendu qu'en ce qui concerne le co-solvant ajouté il ne s'agit pas d'hexaméthylphosphotriamide
(C) éventuellement saponification du composé acide malonique en l'acide correspondant et
(D) décarboxylation finale de l'acide correspondant.

2. Procédé selon la revendication 1, pour la préparation d'acides 3-alkylcarboxyliques optiquement actifs de formule générale (Ia) ou (Ib), 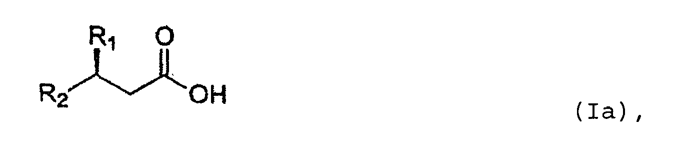 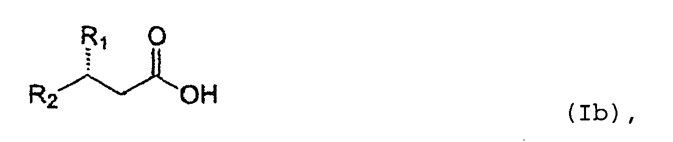 **R¹** et **R²** représentant, indépendamment l'un de l'autre, un radical hydrocarboné en C₁-C₁₅ linéaire ou ramifié, saturé ou insaturé, cyclique ou contenant des groupes cycliques, éventuellement substitué par Q
et Q est choisi dans l'ensemble comportant des atomes d'halogène, des groupes amino, amido, phtalimido, hydroxy, cyano, nitro, alcoxy, aryloxy, aralkyloxy, alkylthio, acyle, silyle, silyloxy, aryle, hétéroaryle,
comportant les étapes suivantes:
A) conversion d'un composé de formule générale (IIa) ou (IIb) 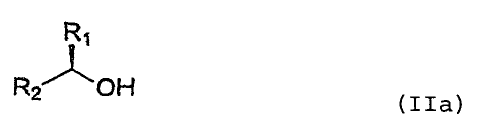 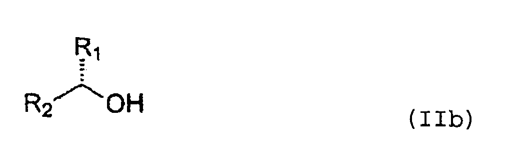 en composés activés de formule générale (IIIa) ou (IIIb) 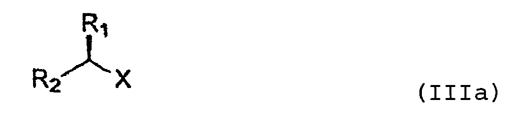 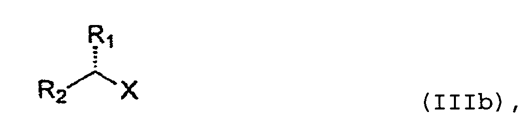 X étant choisi dans l'ensemble consistant en les atomes d'halogène et les groupes OSO₂R, OSO₂OR et OCOR, et
R représentant un atome de fluor ou un radical hydrocarboné en C₁-C₁₅ linéaire ou ramifié, saturé ou insaturé, cyclique ou contenant des groupes cycliques, éventuellement substitué par Q
B) mise en réaction des composés de formule générale (IIIa) ou (IIIb), obtenus selon l'étape A), avec un dérivé d'acide malonique, avec obtention de composés de formule générale (IVa) ou (IVb), la réaction s'effectuant exclusivement en présence d'un ou plusieurs solvants choisis dans la classe des éthers ou des esters d'acides carboxyliques, une addition d'un ou plusieurs solvants polaires aprotiques ou alcools en tant que co-solvant(s) pouvant éventuellement être effectuée, cette addition représentant au maximum 30 % du volume de solvants ajoutés au total, étant entendu qu'en ce qui concerne le co-solvant ajouté il ne s'agit pas d'hexaméthylphosphotriamide, et 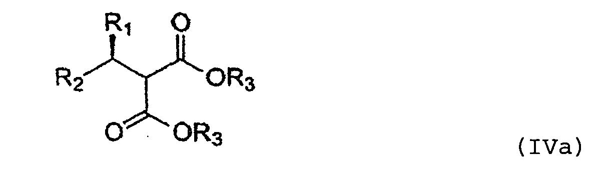 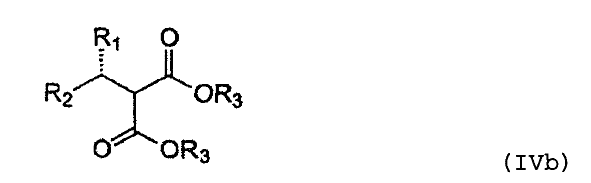 R³ dans la formule générale (IVa) ou (IVb) représentant chacun, indépendamment les uns des autres, un atome d'hydrogène ou un radical hydrocarboné en C₁-C₁₅ linéaire ou ramifié, saturé ou insaturé, cyclique ou contenant des groupes cycliques, un radical trialkyl(C₁-C₂₀)- ou triaryl-(C₁-C₂₀)-silyle,
C) éventuellement saponification des composés de formule générale (IVa) ou (IVb), obtenus selon l'étape B), avec obtention d'acides dicarboxyliques de formule générale (Va) ou (Vb) 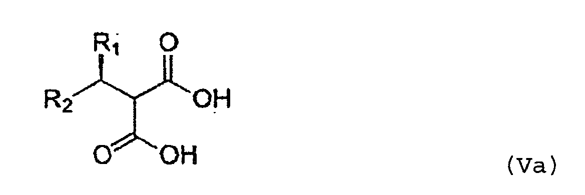 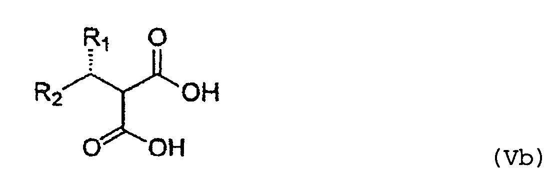
D) et décarboxylation finale de l'acide dicarboxylique de formule générale (Va) ou (Vb) obtenu selon l'étape C).

3. Procédé selon la revendication 1 ou 2, **caractérisé en ce que** dans la réaction selon l'étape B), dans le cas d'une éventuelle addition d'un ou plusieurs solvants aprotiques polaires en tant que co-solvants, ces derniers sont choisis dans la classe des solvants comportant des nitriles, des carboxamides, des dérivés d'urée et des sulfoxydes.

4. Procédé selon la revendication 1 ou 2, **caractérisé en ce que** dans la réaction selon l'étape B), dans le cas d'une éventuelle addition d'un ou plusieurs solvants aprotiques polaires en tant que co-solvants, ces derniers sont choisis dans l'ensemble comportant l'acétonitrile, le propionitrile, le butyronitrile, le formamide, le N-méthylformamide, le diméthylformamide, le diéthylformamide, le diméthylacétamide, le diéthylacétamide, la N-méthylpyrrolidone, la 1,3-diméthyl-3,4,5,6-tétrahydro-2(1H)-pyrimidinone et le diméthylsulfoxyde.

5. Procédé selon la revendication 1 ou 2, **caractérisé en ce que** dans la réaction selon l'étape B), dans le cas d'une éventuelle addition d'alcool en tant que co-solvant, ce dernier est choisi dans l'ensemble comportant le méthanol, l'éthanol, le propanol, l'isopropanol, le butanol, l'isobutanol, le tert-butanol, le pentanol et l'alcool amylique.

6. Procédé selon une ou plusieurs des revendications 1 à 5, **caractérisé en ce que** l'addition d'un ou plusieurs alcools ou solvants protiques polaires en tant que co-solvant(s) représente au maximum 20 % du volume de solvants ajoutés au total.

7. Procédé selon la revendication 1 ou 2, **caractérisé en ce que** la réaction selon l'étape B) est effectuée exclusivement en présence d'un ou plusieurs solvants choisis dans la classe des éthers ou des esters d'acides carboxyliques.

8. Procédé selon la revendication 1 ou 2, **caractérisé en ce que** la réaction selon l'étape B) est effectuée exclusivement en présence d'un ou plusieurs solvants choisis dans la classe des éthers.

9. Procédé selon une ou plusieurs des revendications 1 à 8, **caractérisé en ce que** les composés obtenus selon l'étape C) sont purifiés dans une étape supplémentaire avant la décarboxylation.

10. Procédé selon une ou plusieurs des revendications 1 à 9, **caractérisé en ce que** l'étape B) est effectuée en présence d'un ou plusieurs solvants choisis dans l'ensemble contenant le tétrahydrofuranne, le 2-méthyltétrahydrofuranne, le 2,5-diméthyltétrahydrofuranne, le 2,5-diméthoxytétrahydrofuranne, le 1,4-dioxanne, le diméthoxyéthane, le diéthoxyéthane, l'éther diméthylique de diéthylèneglycol, l'éther diéthylique de diéthylèneglycol, l'éther dipropylique de diéthylèneglycol, l'éther dibutylique de diéthylèneglycol, l'éther diméthylique de triéthylèneglycol, l'éther diéthylique de triéthylèneglycol et l'éther dibutylique de triéthylèneglycol.

11. Procédé selon une ou plusieurs des revendications 1 à 9, **caractérisé en ce que** l'étape B) est effectuée exclusivement en présence d'un ou plusieurs solvants choisis dans l'ensemble contenant le tétrahydrofuranne, le 2-méthyltétrahydrofuranne, le 2,5-diméthyltétrahydrofuranne, le 2,5-diméthoxytétrahydrofuranne, le 1,4-dioxanne, le diméthoxyéthane, le diéthoxyéthane, l'éther diméthylique de diéthylèneglycol, l'éther diéthylique de diéthylèneglycol, l'éther dipropylique de diéthylèneglycol, l'éther dibutylique de diéthylèneglycol, l'éther diméthylique de triéthylèneglycol, l'éther diéthylique de triéthylèneglycol et l'éther dibutylique de triéthylèneglycol et l'acétate d'éthyle, l'acétate de propyle, l'acétate d'isopropyle et l'acétate de butyle.

12. Procédé selon une ou plusieurs des revendications 1 à 11, **caractérisé en ce que** pour l'enrichissement en énantiomère les composés de formule générale (Va) ou (Vb) sont cristallisés ou recristallisés dans une étape supplémentaire.
